(19) Europäisches Patentamt
European Patent Office
Office européen des brevets

(11) EP 4 596 070 A1

(12) **EUROPEAN PATENT APPLICATION**
published in accordance with Art. 153(4) EPC

(43) Date of publication:
06.08.2025 Bulletin 2025/32

(21) Application number: 23872386.0

(22) Date of filing: 27.09.2023

(51) International Patent Classification (IPC):
*A63H 11/00* (2006.01)    *B25J 13/08* (2006.01)

(52) Cooperative Patent Classification (CPC):
A63H 11/00; B25J 13/08

(86) International application number:
PCT/JP2023/035067

(87) International publication number:
WO 2024/071164 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.09.2022 JP 2022156758

(71) Applicant: Nitto Denko Corporation
Ibaraki-shi, Osaka 567-8680 (JP)

(72) Inventors:
• KIYOSHIMA, Keita
Ibaraki-shi, Osaka 567-8680 (JP)
• FUKUSHIMA, Rihito
Ibaraki-shi, Osaka 567-8680 (JP)
• YAMAUCHI, Kengo
Ibaraki-shi, Osaka 567-8680 (JP)
• SHIMIZU, Yusuke
Ibaraki-shi, Osaka 567-8680 (JP)

(74) Representative: Epping - Hermann - Fischer
Patentanwaltsgesellschaft mbH
Schloßschmidstraße 5
80639 München (DE)

(54) **ROBOT, LEARNING DEVICE, CONTROL METHOD, AND PROGRAM**

(57)    Interaction with a user is induced at an appropriate timing. A robot includes an acquisition unit configured to acquire at least one of a captured image of a user or biological information of the user; and an action control unit configured to instruct to perform a predetermined action that induces the interaction with the user in accordance with a state of the user based on the at least one of the captured image or the biological information.

FIG.6

**Description**

TECHNICAL FIELD

The present disclosure relates to a robot, a learning device, a control method, and a program.

BACKGROUND

Conventionally, a robot designed to interact with a user is known. Additionally, a robot configured to provide comfort through interaction with a user is known.

Patent Document 1 discloses that an action mode is changed in a robot configured to measure a body temperature of a user and determine a physical condition based on a body temperature cycle, in response to determining the timing that requires consideration by referring to a menstrual cycle of a woman.

Related Art Document

Patent Document

[Patent Document 1] Japanese Laid-open Patent Application Publication No. 2009-104878

SUMMARY OF THE INVENTION

Problem to be solved by the invention

However, in Patent Document 1, the timing that requires consideration is limited because a body temperature cycle of a user is assumed. It is difficult to determine, based on a body temperature cycle in various daily situations, the timing for engagement with the user without causing stress to the user. If the user is engaged at an inappropriate timing, it does not lead to interaction with the user, and comfort cannot be provided.

The disclosed technique aims to induce interaction with a user at an appropriate timing.

Means for Solving the Problem

An aspect of the present disclosure is a robot including an acquisition unit configured to acquire at least one of a captured image of a user or biological information of the user, and an action control unit configured to instruct to perform a predetermined action that induces interaction with the user in accordance with a state of the user based on the at least one of the captured image or the biological information.

Another aspect of the present disclosure is a learning device communicably connected to a robot. The learning device includes a state observation unit configured to observe, based on at least one of a captured image of a user or biological information of the user, a state of the user, and a learning unit configured to generate a learning model by machine learning. The learning model is configured to receive the state of the user and output a value of an action of the robot.

Effect of the invention

According to an aspect of the present disclosure, interaction with a user can be induced at an appropriate timing.

BRIEF DESCRIPTION OF THE DRAWINGS

[FIG. 1] FIG. 1 is a perspective view of a robot according to an embodiment.
[FIG. 2] FIG. 2 is a side view of the robot according to the embodiment.
[FIG. 3] FIG. 3 is a cross-sectional view of the robot of the embodiment along a cutting line III-III.
[FIG. 4] FIG. 4 is a diagram illustrating a configuration of a vital sensor according to the embodiment.
[FIG. 5] FIG. 5 is a block diagram illustrating a hardware configuration of a controller according to the embodiment.
[FIG. 6] FIG. 6 is a block diagram illustrating a functional configuration of the controller according to the embodiment.
[FIG. 7] FIG. 7 is a block diagram illustrating a hardware configuration of an estimation unit (a learning device) according to the embodiment.

[FIG. 8] FIG. 8 is a block diagram illustrating a functional configuration of the estimation unit (the learning device) according to the embodiment.

[FIG. 9] FIG. 9 is a schematic diagram of a learning model of a neuron according to the embodiment.

[FIG. 10] FIG. 10 is a schematic diagram of a learning model of a neural network according to the embodiment.

[FIG. 11] FIG. 11 is a flowchart illustrating a process of the controller according to the embodiment.

[FIG. 12] FIG. 12 is a flowchart illustrating a process of the estimation unit (the learning device) of the embodiment.

[FIG. 13] FIG. 13 is a block diagram illustrating a functional configuration of an estimation unit (a learning device) of a modification example.

[FIG. 14] FIG. 14 is a flowchart illustrating a process of the estimation unit (the learning device) of the modification example.

DESCRIPTION OF THE EMBODIMENTS

In the following, embodiments of the present disclosure will be described in detail with reference to the drawings. In the drawings, the same components are denoted by the same reference symbols, and duplicated description will be appropriately omitted.

The embodiments described below are examples of a robot for embodying the technical idea of the present disclosure, and the present disclosure is not limited to the embodiments described below. The dimensions, materials, shapes, relative arrangements, and the like of the components described below are intended to illustrate the examples, not to limit the scope of the present disclosure thereto, unless otherwise specified. Additionally, the size, positional relationship, and the like of members illustrated in the drawings may be exaggerated for clarity of description.

<Overall Configuration Example of Robot 100>

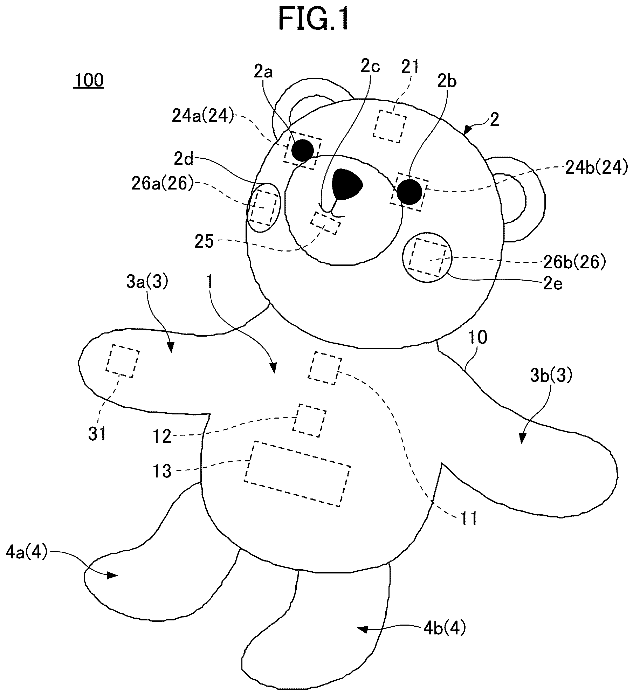

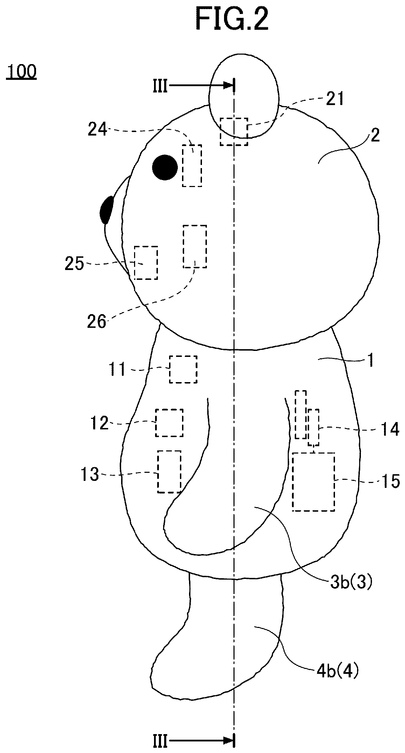

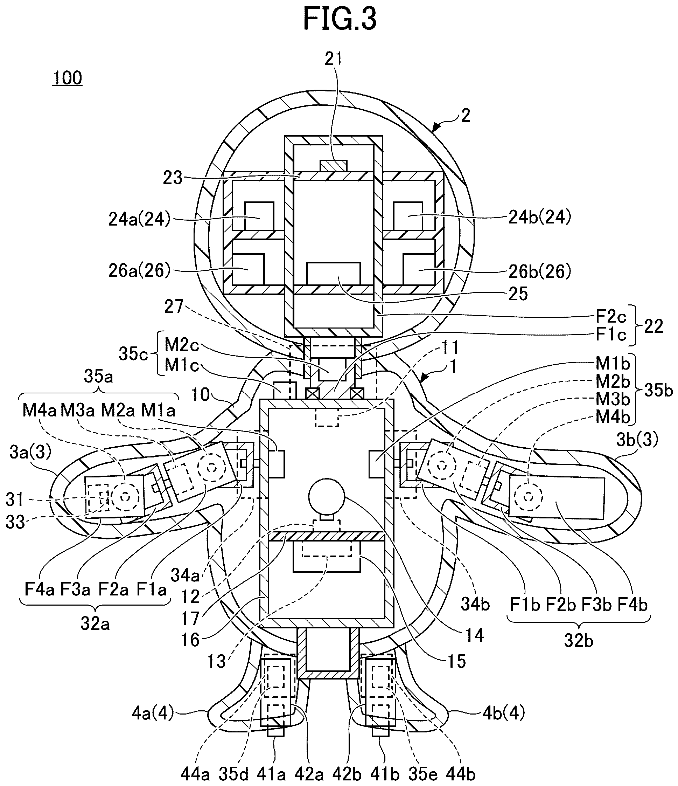

A configuration of a robot 100 according to an embodiment will be described with reference to FIGS. 1 to 3. FIG. 1 is a perspective view illustrating the robot 100 according to the embodiment. FIG. 2 is a side view of the robot 100. FIG. 3 is a cross-sectional view along a cutting line III-III in FIG. 2.

The robot 100 is a robot that includes exterior members 10 and that is configured to be driven by supplied power. The robot 100 described in the present embodiment as an example is a doll-type communication robot in the shape of a bear cub. The robot 100 is manufactured to have a size and weight suitable for being held by a user. Here, the user indicates a user of the robot 100. Typical examples of the user include a working adult living alone, a senior person whose child has moved out, and a frail elderly person who is a target of home healthcare. Additionally, the user may include a contact person who simply contacts the robot 100, such as an administrator of the robot 100, in addition to the user of the robot 100.

The exterior member 10 has flexibility. The exterior member 10 contains, for example, a soft material that is comfortable to touch when the user of the robot 100 touches the robot 100. As the material of the exterior member 10, a material containing an organic material, such as urethane foam, rubber, resin, or fiber can be used. The exterior member 10 preferably includes an exterior, such as a urethane foam material, having a heat insulating property, and a soft cloth material covering the outer surface of the exterior.

The robot 100 includes, for example, a trunk 1, a head 2, arms 3, and legs 4. The head 2 has a right eye 2a, a left eye 2b, a mouth 2c, a right cheek 2d, and a left cheek 2e. The arms 3 include a right arm 3a and a left arm 3b, and the legs 4 include a right leg 4a and a left leg 4b. Here, the trunk 1 corresponds to a robot main body. Each of the head 2, the arm 3, and the leg 4 corresponds to a driving body that is connected to the robot main body so as to be relatively displaceable.

In the present embodiment, the arm 3 is configured to be displaceable with respect to the trunk 1. For example, when the robot 100 is held by the user, the robot 100 displaces the right arm 3a and the left arm 3b to contact a neck, a trunk, or the like of the user so as to hold the user. This operation allows the user to feel an affinity for the robot 100, and thus promotes the interaction between the user and the robot 100. Here, the interaction with the user indicates an action of the user and the robot 100 touching each other (a contact action), such as rubbing, tapping (touching), hugging (embracing), and the like.

The trunk 1, the head 2, the arms 3, and the legs 4 are all covered with the exterior members 10. The exterior member at the trunk 1 and the exterior member at the arm 3 are integrated, and the exterior members at the head 2 and the leg 4 are separated from the exterior member at the trunk 1 and the arm 3. However, the embodiments are not limited to these configurations, and for example, only a portion of the robot 100 that is likely to be contacted by the user may be covered by the exterior member 10. Additionally, at least one of the exterior members 10 at the trunk 1, the head 2, the arm 3, and the leg 4 may be separated from the other exterior members. Further, a portion in the head 2, the arm 3, and the leg 4 that does not displace may be configured only by the exterior member 10 without including a component such as a sensor therein.

The robot 100 includes a camera 11, a tactile sensor 12, a controller 13, a vital sensor 14, a battery 15, a first capacitive sensor 21, and a second capacitive sensor 31 inside the exterior members 10. Additionally, the robot 100 includes the camera 11, the tactile sensor 12, the controller 13, the vital sensor 14, and the battery 15 inside the exterior member 10 at the trunk 1. Additionally, the robot 100 includes the first capacitive sensor 21 inside the exterior member 10 at the head 2, and the second capacitive sensor 31 inside the exterior member 10 at the arm 3.

Additionally, the robot 100 includes displays 24, a speaker 25, and lights 26 inside the exterior member 10 at the head 2. Additionally, the robot 100 includes the displays 24 inside the exterior member 10 at the right eye 2a and the left eye 2b. Additionally, the robot 100 includes the speaker 25 inside the exterior member 10 at the mouth 2c, and the lights 26 inside the exterior member 10 at the right cheek 2d and the left cheek 2e.

More specifically, as illustrated in FIG. 3, the robot 100 includes a trunk frame 16 and a trunk mounting base 17 inside the exterior member 10 at the trunk 1. Additionally, the robot 100 includes a head frame 22 and a head mounting base 23 inside the exterior member 10 at the head 2. Further, the robot 100 includes a right arm frame 32a and a right arm mounting base 33 inside the exterior member 10 at the right arm 3a, and a left arm frame 32b inside the exterior member 10 at the left arm 3b. In addition, the robot 100 includes a right leg frame 42a inside the exterior member 10 at the right leg 4a, and a left leg frame 42b inside the exterior member 10 at the left leg 4b.

The trunk frame 16, the head frame 22, the right arm frame 32a, the left arm frame 32b, the right leg frame 42a, and the left leg frame 42b are structures each formed by combining multiple columnar members. The trunk mounting base 17, the head mounting base 23, and the right arm mounting base 33 are plate members having placement surfaces. The trunk mounting base 17 is fixed to the trunk frame 16, the head mounting base 23 is fixed to the head frame 22, and the right arm mounting base 33 is fixed to the right arm frame 32a. Here, the trunk frame 16, the head frame 22, the right arm frame 32a, the left arm frame 32b, the right leg frame 42a, and the left leg frame 42b may be formed in a box shape including multiple plate members.

The right arm frame 32a is connected to the trunk frame 16 via a right arm connection mechanism 34a, and is driven by a right arm servo motor 35a, thereby being relatively displaceable with respect to the trunk frame 16. The displacement of the right arm frame 32a causes the right arm 3a to displace relative to the trunk 1. The right arm connection mechanism 34a preferably includes a reduction gear that increases the output torque of the right arm servo motor 35a, for example.

In the present embodiment, the right arm frame 32a is configured by a multi-joint robot arm including multiple frame members and multiple connection mechanisms. For example, the right arm frame 32a includes a right shoulder frame F1a, a right upper arm frame F2a, a right elbow frame F3a, and a right forearm frame F4a. The trunk frame 16, the right shoulder frame F1a, the right upper arm frame F2a, the right elbow frame F3a, and the right forearm frame F4a are connected to each other via respective connection mechanisms.

The right arm servo motor 35a is a generic term of multiple servo motors. For example, the right arm servo motor 35a includes a right shoulder servo motor M1a, a right upper arm servo motor M2a, a right elbow servo motor M3a, and a right forearm servo motor M4a. The right shoulder servo motor M1a rotates the right shoulder frame F1a about a rotation axis perpendicular to the trunk frame 16. The right upper arm servo motor M2a rotates the right upper arm frame F2a about a rotation axis perpendicular to a rotation axis of the right shoulder frame F1a. The right elbow servo motor M3a rotates the right elbow frame F3a about a rotation axis perpendicular to a rotation axis of the right upper arm frame F2a. The right forearm servo motor M4a rotates the right forearm frame F4a about a rotation axis perpendicular to a rotation axis of the right elbow frame F3a.

The left arm frame 32b is connected to the trunk frame 16 via a left arm connection mechanism 34b, and is driven by a left arm servo motor 35b, thereby being relatively displaceable with respect to the trunk frame 16. The displacement of the left arm frame 32b causes the left arm 3b to displace relative to the trunk 1. The left arm connection mechanism 34b preferably includes a reduction gear that increases the output torque of the left arm servo motor 35b, for example.

In the present embodiment, the left arm frame 32b is configured by a multi-joint robot arm including multiple frame members and multiple connection mechanisms. For example, the left arm frame 32b includes a left shoulder frame F1b, a left upper arm frame F2b, a left elbow frame F3b, and a left forearm frame F4b. The trunk frame 16, the left shoulder frame F1b, the left upper arm frame F2b, the left elbow frame F3b, and the left forearm frame F4b are connected to each other via respective connection mechanisms.

The left arm servo motor 35b is a generic term of multiple servo motors. For example, the left arm servo motor 35b includes a left shoulder servo motor M1b, a left upper arm servo motor M2b, a left elbow servo motor M3b, and a left forearm servo motor M4b. The left shoulder servo motor M1b rotates the left shoulder frame F1b about a rotation axis perpendicular to the trunk frame 16. The left upper arm servo motor M2b rotates the left upper arm frame F2b about a rotation axis perpendicular to a rotation axis of the left shoulder frame F1b. The left elbow servo motor M3b rotates the left elbow frame F3b about a rotation axis perpendicular to a rotation axis of the left upper arm frame F2b. The left forearm servo motor M4b rotates the left forearm frame F4b about a rotation axis perpendicular to a rotation axis of the left elbow frame F3b.

The arm 3 includes the four-axis joints as described above, thereby enabling the robot 100 to realize a more realistic operation. For example, the robot 100 can perform an action that induces interaction with the user by moving the arms 3 to "spread arms" toward the user who has a neutral emotion and is doing nothing. Additionally, the robot 100 can perform an action that induces interaction with the user by shaking the arm 3 to "be frightened" toward the user who is walking with an angry emotion.

The head frame 22 is connected to the trunk frame 16 via a head connection mechanism 27, and is driven by a head servo motor 35c, thereby being relatively displaceable with respect to the trunk frame 16. The displacement of the head frame 22 causes the head 2 to displace relative to the trunk 1. The head connection mechanism 27 preferably includes a reduction gear that increases the output torque of the head servo motor 35c, for example.

In the present embodiment, the head frame 22 includes a neck frame F1c and a face frame F2c. The trunk frame 16, the neck frame F1c, and the face frame F2c are connected to each other via respective connection mechanisms.

The head servo motor 35c is a generic term of multiple servo motors. For example, the head servo motor 35c includes a neck servo motor M1c and a face servo motor M2c. The neck servo motor M1c rotates the neck frame F1c about a rotation axis perpendicular to the trunk frame 16. The face servo motor M2c rotates the face frame F2c around a rotation axis perpendicular to a rotation axis of the neck frame F1c.

The head 2 includes the two-axis joints as described above, thereby enabling the robot 100 to realize a more realistic operation. For example, the robot 100 can perform an action that induces interaction with the user by demonstrating an emotion of worrying about the user by moving the head 2 to "look up" (pay attention to) the user who operates a mobile phone with a feeling of disgust.

The right leg frame 42a is connected to the trunk frame 16 via a right leg connection mechanism 44a, and includes a right leg wheel 41a on the bottom side. In order to stabilize the posture of the robot 100, the robot 100 preferably includes two right leg wheels 41a in the front-rear direction of the right leg frame 42a. The right leg wheel 41a is driven by a right leg servo motor 35d, thereby being rotatable about a rotation axis perpendicular to the front-rear direction of the right leg frame 42a. The rotation of the right leg wheel 41a enables the robot 100 to travel. The right leg connection mechanism 44a preferably includes a reduction gear that increases the output torque of the right leg servo motor 35d, for example.

The left leg frame 42b is connected to the trunk frame 16 via a left leg connection mechanism 44b, and includes a left leg wheel 41b on the bottom side. In order to stabilize the posture of the robot 100, the robot 100 preferably includes two left leg wheels 41b in the front-rear direction of the left leg frame 42b. The left leg wheel 41b is driven by the left leg servo motor 35e, thereby being rotatable about a rotation axis perpendicular to the front-rear direction of the left leg frame 42b. The rotation of the left leg wheel 41b enables the robot 100 to travel. The left leg connection mechanism 44b preferably includes a reduction gear that increases the output torque of a left leg servo motor 35e, for example.

In the present embodiment, the robot 100 moves forward or backward by simultaneously rotating the right leg wheel 41a and the left leg wheel 41b forward or backward. The robot 100 turns right or left by braking one of the right leg wheel 41a or the left leg wheel 41b with a brake and turning the other forward or backward.

As described, the robot 100 can realize a more realistic operation by the leg 4. For example, the robot 100 can perform an action that induces interaction with the user by moving the leg 4 to "approach" the user who is standing still with feelings of sadness or surprise.

The camera 11 is fixed to the trunk frame 16. The tactile sensor 12, the controller 13, the vital sensor 14, and the battery 15 are fixed to the trunk mounting base 17. The controller 13 and the battery 15 are fixed to the side of the trunk mounting base 17 opposite to the side to which the tactile sensor 12 and the vital sensor 14 are fixed. Here, the arrangement of the controller 13 and the battery 15 is due to the space available on the trunk mounting base 17 and is not limited to the above described arrangement. However, if the battery 15 is fixed to the side of the trunk mounting base 17 opposite to the side to which the tactile sensor 12 and the vital sensor 14 are fixed, the center of gravity of the robot 100 is lowered because the battery 15 is heavier than other components. The center of gravity of the robot 100 is preferably low because at least one of the position or the posture of the robot 100 is stabilized and at least one of charging or replacement of the battery 15 is easily performed.

The first capacitive sensor 21 is fixed to the head mounting base 23, and the second capacitive sensor 31 is fixed to the right arm mounting base 33. The display 24 includes a right-eye display 24a and a left-eye display 24b. The right-eye display 24a, the left-eye display 24b, and the speaker 25 are fixed to the head frame 22. The lights 26 include a right-cheek light 26a and a left-cheek light 26b. The right-cheek light 26a and the left-cheek light 26b are fixed to the head frame 22.

Here, the camera 11, the tactile sensor 12, the controller 13, the vital sensor 14, the battery 15, the first capacitive sensor 21, the second capacitive sensor 31, and the like can be fixed by a screw member, an adhesive member, or the like. Additionally, the right-eye display 24a, the left-eye display 24b, the speaker 25, the right-cheek light 26a, the left-cheek light 26b, and the like can also be fixed by screw members, adhesive members, or the like.

The materials of the trunk frame 16, the trunk mounting base 17, the head frame 22, the head mounting base 23, the right arm frame 32a, the right arm mounting base 33, and the left arm frame 32b are not particularly limited, and a resin material, a metallic material, or the like can be used. However, from the viewpoint of ensuring strength during driving, it is preferable to use a metallic material, such as aluminum, for the trunk frame 16, the right arm frame 32a, and the left arm frame 32b. If the strength can be obtained, it is preferable to use a resin material for the material of each of these parts in order to reduce the weight of the robot 100. The materials of the trunk mounting base 17, the head frame 22, the head mounting base 23, the right arm mounting base 33, and the left arm frame 32b are not particularly limited, and a resin material or a metallic material can be used. However, from the viewpoint of reducing the weight of the robot 100, it is preferable to use a resin material.

The controller 13 is communicably connected to each of the camera 11, the tactile sensor 12, the vital sensor 14, the first capacitive sensor 21, the second capacitive sensor 31, the right arm servo motor 35a, and the left arm servo motor 35b by wire or wirelessly. Additionally, the controller 13 is communicably connected to each of the head servo motor 35c, the right leg servo motor 35d, and the left leg servo motor 35e by wire or wirelessly. Further, the controller 13 is communicably connected to each of the right-eye display 24a, the left-eye display 24b, the speaker 25, the right-cheek light 26a, and the left-cheek light 26b by wire or wirelessly.

The camera 11 is an image sensor configured to output a captured image of the surroundings of the robot 100 to the controller 13. In the present embodiment, the camera 11 is an example of an imaging section configured to image a user. The camera 11 includes a lens and an imaging element that captures an image formed by the lens. As the imaging element, a charge coupled device (CCD), a complementary metal-oxide semiconductor (CMOS), or the like can be used. The captured image may be either a still image or a moving image.

Additionally, the camera 11 is preferably configured by a time-of-flight (TOF) camera configured to output a distance image of the surroundings of the robot 100 to the controller 13. Therefore, the captured image output from the camera 11 may include a three-dimensional captured image (the distance image) in addition to the two-dimensional captured image or instead of the two-dimensional captured image. The captured image is used for detection of the presence or approach of the user, detection of the distance from the robot 100 to the user, authentication of the user, estimation of emotion or an action of the user, or the like. The captured image is an example of a captured image of the user. Additionally, the robot 100 may include, in addition to the camera 11, a human presence sensor, such as an ultrasonic sensor, an infrared sensor, a millimeter wave radar, or a light detection and raging (LiDAR).

The tactile sensor 12 is a sensor element configured to detect information felt by a tactile sense inherent in a human hand or the like, convert the information into a tactile signal, which is an electrical signal, and output the tactile signal to the controller 13. For example, the tactile sensor 12 converts information on pressure or vibration generated by the user contacting the robot 100 into a tactile signal by a piezoelectric element, and outputs the tactile signal to the controller 13. The tactile signal output from the tactile sensor 12 is used to detect whether a user 200 has contacted the robot 100 or is present.

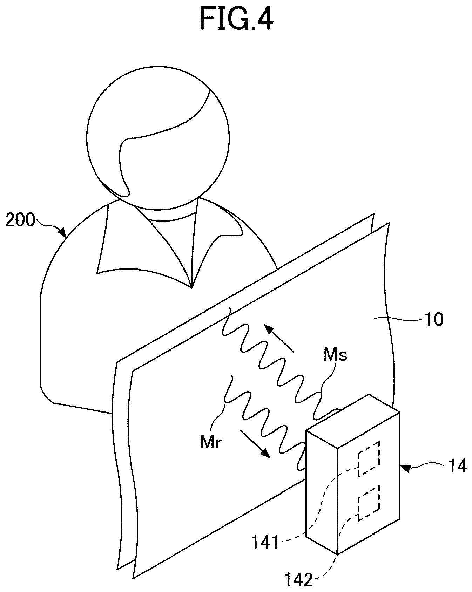

The vital sensor 14 is an example of an electromagnetic wave sensor configured to acquire biological information of the user by using electromagnetic waves. The vital sensor 14 will be described in detail later with reference to FIG. 4.

The first capacitive sensor 21 and the second capacitive sensor 31 are sensor elements configured to output, to the controller 13, a capacitance signal obtained by detecting, based on a change in capacitance, that the user has come into contact with or come in proximity to the robot 100. The first capacitive sensor 21 is preferably a rigid sensor having no flexibility in terms of stabilization of the exterior member 10. The arm 3 is a part that is easily touched by the user, and thus the second capacitive sensor 31 is preferably a sensor having flexibility including a conductive thread or the like from the viewpoint of improving the touch feeling. The capacitance signals output from the first capacitive sensor 21 and the second capacitive sensor 31 are used to detect that the user has come in proximity to the robot 100 or is present.

The right-eye display 24a and the left-eye display 24b are display modules configured to display character strings, such as characters, numerals, and symbols, or images in response to a command from the controller 13. The right-eye display 24a and the left-eye display 24b are configured by, for example, liquid crystal display modules. The character strings or images displayed on the right-eye display 24a and the left-eye display 24b are used to express the emotion of the robot 100. For example, the robot 100 displays "smiling" images on the right-eye display 24a and the left-eye display 24b toward the user who is sitting with feelings of happiness to empathize with the happiness, thereby implicitly inducing interaction with the user.

The speaker 25 is a speaker unit configured to amplify an audio signal from the controller 13 and emit sound. The sound emitted from the speaker 25 is a word or a call of the robot 100, and is used to express the emotion of the robot 100. For example, the robot 100 can perform an action that induces interaction with the user by emitting a sound "outputting a (worried) voice" from the speaker 25 to the user who is doing housework with feelings of sadness.

The right-cheek light 26a and the left-cheek light 26b are light modules configured to blink or change colors in response to an on/off signal from the controller 13. The right-cheek light 26a and the left-cheek light 26b are configured by, for example, light emitting diodes (LEDs). The blinking or the changing of the colors of the right-cheek light 26a and the left-cheek light 26b is used to express the emotion of the robot 100. For example, the robot 100 can perform an action that induces interaction with the user by causing the right-cheek light 26a and the left-cheek light 26b to blink in blue for the user who is sitting with feelings of sadness to demonstrate empathy.

The battery 15 is a power supply configured to supply power to each of the camera 11, the tactile sensor 12, the controller 13, the vital sensor 14, the first capacitive sensor 21, the second capacitive sensor 31, the right arm servo motor 35a, and the left arm servo motor 35b. Additionally, the battery 15 supplies power to each of the head servo motor 35c, the right leg servo motor 35d, and the left leg servo motor 35e. Further, the battery 15 supplies power to each of the right-eye display 24a, the left-eye display 24b, the speaker 25, the right-cheek light 26a, and the left-cheek light 26b. Various secondary batteries, such as a lithium ion battery and a lithium polymer battery can be used as the battery 15.

Here, the various sensors, such as the first capacitive sensor 21, and the second capacitive sensor 31 in the robot 100 are not essential components. The robot 100 may only include at least the camera 11, the vital sensor 14, and the tactile sensor 12. The installation positions of these components can also be changed as appropriate. Further, the various sensors, such as the camera 11, the vital sensor 14, and the tactile sensor 12 may be disposed outside the robot 100 and transmit necessary information to the robot 100 or an external device via wireless connection. For example, a learning device configured by a personal computer (PC) or a server is an example of the external device.

Additionally, the robot 100 does not necessarily include the controller 13 inside the exterior member 10, and the controller 13 can communicate with each device from the outside of the exterior member 10 via wireless connection. The battery 15 can supply power to each component from the outside of the exterior member 10.

In the present embodiment, a configuration in which the head 2, the arm 3, and the leg 4 are displaceable is described as an example, but the present embodiment is not limited thereto, and at least one of the head 2, the arm 3, or the leg 4 may be displaceable. Additionally, the arm 3 is configured by a four-axis multi-joint robot arm, but may be configured by a six-axis multi-joint robot arm. Further, the arm 3 is preferably connectable to an end effector, such as a hand. Additionally, the leg 4 is configured by a wheel system, but can be configured by a crawler system, a leg system, or the like.

The configuration and shape of the robot 100 are not limited to those examples described in the present embodiment, and can be appropriately changed according to the preference of the user, the use form of the robot 100, and the like. For example, the robot 100 may be in a form of a robot arm, such as an industrial robot, or a form of a human, such as a humanoid, instead of a form of imitating a bear cub. Additionally, the robot 100 may be in a form of a mobile device, such as a drone or a vehicle, including at least one of an arm, a display, a speaker, a light, and the like.

<Configuration Example of Vital Sensor 14>

FIG. 4 is a diagram illustrating a configuration of the vital sensor 14. The vital sensor 14 is a microwave Doppler sensor including a microwave transmitter 141 and a microwave receiver 142. The microwave is an example of the electromagnetic wave.

The vital sensor 14 transmits a transmitted wave Ms, which is a microwave, toward the user 200 from the inside of the exterior member 10 of the robot 100 by the microwave transmitter 141. The vital sensor 14 receives, by the microwave receiver 142, a reflected wave Mr of the transmitted wave Ms that is reflected by the user 200.

The vital sensor 14 detects, in a non-contact manner, a minute displacement generated on a body surface due to the beating of the heart of the user 200 or the like, based on a difference between the frequency of the transmitted wave Ms and the frequency of the reflected wave Mr, by using the Doppler effect. The vital sensor 14 can acquire information, such as a heartbeat, respiration, a pulse wave, and a blood pressure, as the biological information of the user 200 based on the detected minute displacement, and output the acquired biological information to the controller 13.

However, the vital sensor 14 is not limited to the microwave Doppler sensor, and may be a sensor configured to detect a minute displacement generated on a body surface by using a change in coupling between a human body and an antenna, or may be a sensor configured to use an electromagnetic wave other than the microwave, such as near-infrared light. Additionally, the vital sensor 14 may be a millimeter wave radar, a microwave radar, or the like. Further, the vital sensor 14 preferably includes a non-contact thermometer configured to detect infrared rays or the like transmitted from the user 200 in addition to the Doppler sensor. In this case, the vital sensor 14 detects biological information of the user 200 including information on at least one of a heartbeat (a pulse), respiration, a blood pressure, or a body temperature.

In the present embodiment, the vital sensor 14 is provided inside the exterior member 10, and thus the user 200 cannot visually recognize the vital sensor 14. This suppresses the user 200 from feeling resistance to the detection of the biological information, and enables the biological information to be smoothly acquired. Additionally, the vital sensor 14 can acquire the biological information in a non-contact manner, and thus can acquire the biological information even when the user moves to some extent, unlike a contact sensor that requires the user to be in contact with the same place for a certain period of time.

Additionally, by promoting the interaction between the user 200 and the robot 100 by the hug operation of the robot 100 or the like, the robot 100 is held by the user 200 and can acquire the biological information in a state of being in contact with or in proximity to the user 200. With this, the robot 100 can acquire highly reliable biological information in which noise is suppressed.

<Configuration Example of Controller 13> (Hardware Configuration Example)

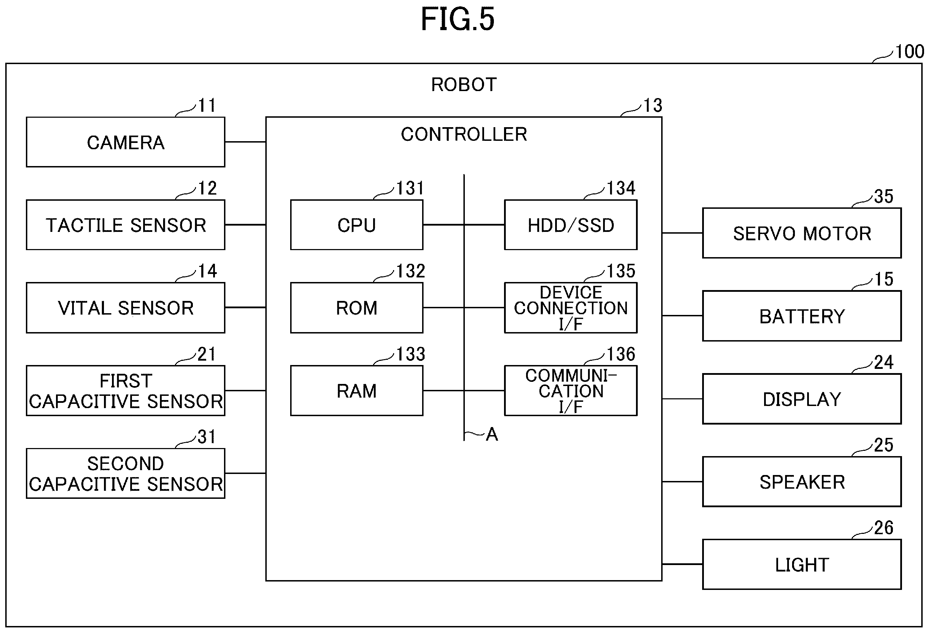

FIG. 5 is a block diagram illustrating a hardware configuration of the controller 13. The controller 13 is constructed by a computer, and includes a central processing unit (CPU) 131, a read only memory (ROM) 132, and a random access memory (RAM) 133. Additionally, the controller 13 includes a hard disk drive/solid state drive (HDD/SSD) 134, a device connection interface (I/F) 135, and a communication I/F 136. These are communicably connected to each other via a system bus A.

The CPU 131 executes control processing including various arithmetic processing. The ROM 132 stores a program used to drive the CPU 131, such as an initial program loader (IPL). The RAM 133 is used as a work area of the CPU 131. The HDD/SSD 134 stores various information, such as programs, captured images acquired by the camera 11, detection information by various sensors, such as the biological information acquired by the vital sensor 14, and tactile information acquired by the tactile sensor 12, and the like.

The device connection I/F 135 is an interface for connecting the controller 13 to various external devices. The external devices herein are the camera 11, the tactile sensor 12, the vital sensor 14, the first capacitive sensor 21, the second capacitive sensor 31, a servo motor 35, the battery 15, and the like. Additionally, the external devices include the display 24, the speaker 25, the light 26, and the like.

Here, the servo motor 35 is a generic term of the right arm servo motor 35a, the left arm servo motor 35b, the head servo motor 35c, the right leg servo motor 35d, and the left leg servo motor 35e. Additionally, the display 24 is a generic term for the right-eye display 24a and the left-eye display 24b. Further, the light 26 is a generic term for the right-cheek light 26a and the left-cheek light 26b.

The communication I/F 136 is an interface for communicating with an external device via a communication network or the like. For example, the controller 13 is connected to the Internet via the communication I/F 136 and communicates with the external device via the Internet.

Here, at least some of the functions realized by the CPU 131 may be realized by an electric circuit or an electronic circuit.

(Functional Configuration Example)

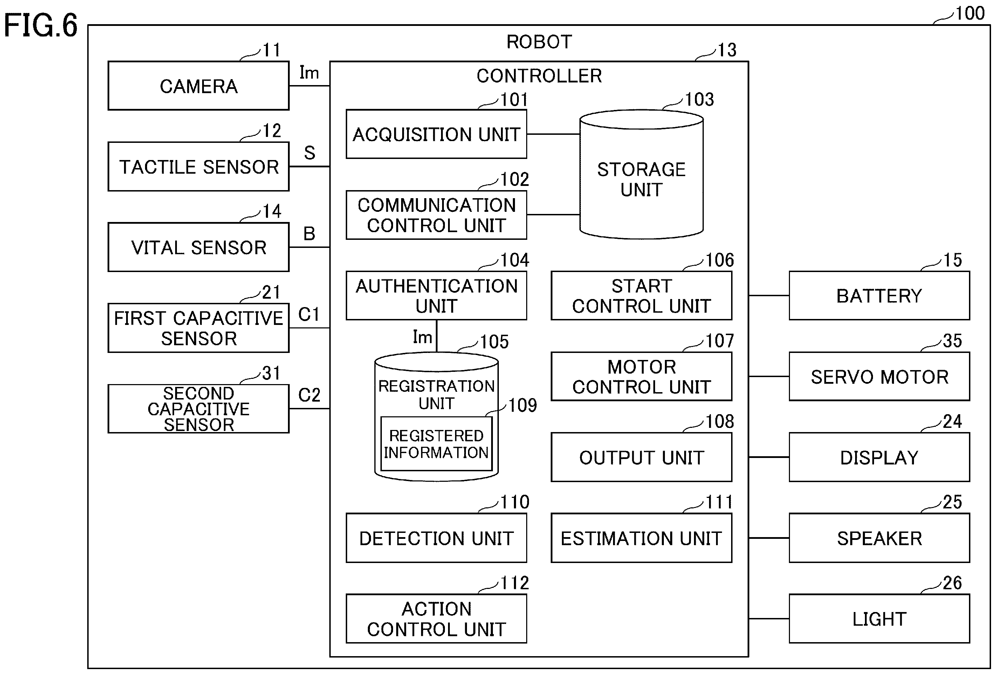

FIG. 6 is a block diagram illustrating a functional configuration of the controller 13. The controller 13 includes an acquisition unit 101, a communication control unit 102, a storage unit 103, an authentication unit 104, a registration unit 105, a start control unit 106, a motor control unit 107, and an output unit 108. Further, the controller 13 includes a detection unit 110, an estimation unit 111, and an action control unit 112.

The controller 13 can realize the functions of the acquisition unit 101 and the output unit 108 by the device connection I/F 135 or the like, and can realize the function of the communication control unit 102 by the communication I/F 136 or the like. Additionally, the controller 13 can realize the functions of the storage unit 103 and the registration unit 105 by a non-volatile memory, such as the HDD/SSD 134. Further, the functions of the authentication unit 104, the start control unit 106, and the motor control unit 107 can be realized by a processor, such as the CPU 131, executing processing defined in a program stored in a non-volatile memory, such as the ROM 132.

Additionally, the functions of the detection unit 110, the estimation unit 111, and the action control unit 112 can be realized by a processor, such as the CPU 131, executing processing defined in a program stored in a non-volatile memory, such as the ROM 132. Here, some of the above-described functions of the controller 13 may be realized by an external device, such as a PC or a server, or may be realized by distributed processing between the controller 13 and the external device. For example, the estimation unit 111 may be configured as a learning device communicably connected to the robot 100.

The acquisition unit 101 controls communication between the controller 13 and the camera 11 to acquire a captured image Im of the user 200 from the camera 11. Additionally, the acquisition unit 101 controls communication between the controller 13 and the tactile sensor 12 to acquire a tactile signal S from the tactile sensor 12. Further, the acquisition unit 101 controls communication between the controller 13 and the vital sensor 14 to acquire biological information B of the user 200 from the vital sensor 14.

Additionally, the acquisition unit 101 controls communication between the controller 13 and the first capacitive sensor 21 to acquire a first capacitance signal C1 from the first capacitive sensor 21. Additionally, the acquisition unit 101 controls communication between the controller 13 and the second capacitive sensor 31 to acquire a second capacitance signal C2 from the second capacitive sensor 31.

The communication control unit 102 controls communication with an external device via a communication network or the like. For example, the communication control unit 102 can transmit the captured image Im acquired by the camera 11, the biological information B acquired by the vital sensor 14, the tactile signal S acquired by the tactile sensor 12, and the like to the external device (for example, a learning device described later) via the communication network.

The storage unit 103 stores the biological information B acquired by the vital sensor 14. The storage unit 103 continuously stores the acquired biological information B while the acquisition unit 101 is acquiring the biological information B from the vital sensor 14. Additionally, the storage unit 103 can store information obtained from the captured image Im by the camera 11, the tactile signal S from the tactile sensor 12, the first capacitance signal C1 from the first capacitive sensor 21, and the second capacitance signal C2 from the second capacitive sensor 31.

The authentication unit 104 performs personal authentication of the user 200 based on the captured image Im of the user 200 captured by the camera 11. For example, the authentication unit 104 performs face authentication by referring to registered information 109 of a face image registered in advance in the registration unit 105, based on the captured image Im including the face of the user 200 captured by the camera 11. With this, the user 200 currently in contact with or in proximity to the robot 100 can be associated with the personal information registered in advance, and the biological information B acquired by the vital sensor 14 can be associated with the personal information. Additionally, the controller 13 can also perform control so as to stop the start of the acquisition of the biological information by the vital sensor 14 when the face image included in the captured image Im is not registered in the registration unit 105.

The start control unit 106 causes the vital sensor 14 to start acquiring the biological information B. For example, when the contact or proximity of the user 200 with respect to the robot 100 is detected by the detection unit 110, the start control unit 106 turns on a switch or the like for supplying power from the battery 15 to the vital sensor 14. With this, the start control unit 106 causes the vital sensor 14 to start acquiring the biological information B.

The detection unit 110 detects the presence or approach of the user 200 around the robot 100 based on the captured image Im and the like obtained by the camera 11. The detection unit 110 preferably detects the distance from the robot 100 to the user 200 based on the captured image Im (the distance image) obtained by the camera 11. Additionally, the detection unit 110 may detect the proximity or presence of the user 200 with respect to the robot 100 based on the first capacitance signal C1 or the second capacitance signal C2. Further, the detection unit 110 detects contact or presence of the user 200 with respect to the robot 100 based on the tactile signal S from the tactile sensor 12.

The estimation unit 111 estimates a predetermined action an (n is an identification number of the action a) of the robot 100 suitable for the state of the emotion of the user 200, based on the captured image Im of the user 200 and the biological information B of the user 200. In the present embodiment, the estimation unit 111 estimates the action at (t is time) of the robot 100 suitable for the state st (t is time) of the user 200 by performing reinforcement learning. However, the estimation unit 111 may estimate the predetermined action at of the robot 100 suitable for the state st of the user 200 by performing other machine learning, such as supervised learning, semi-supervised learning, or unsupervised learning.

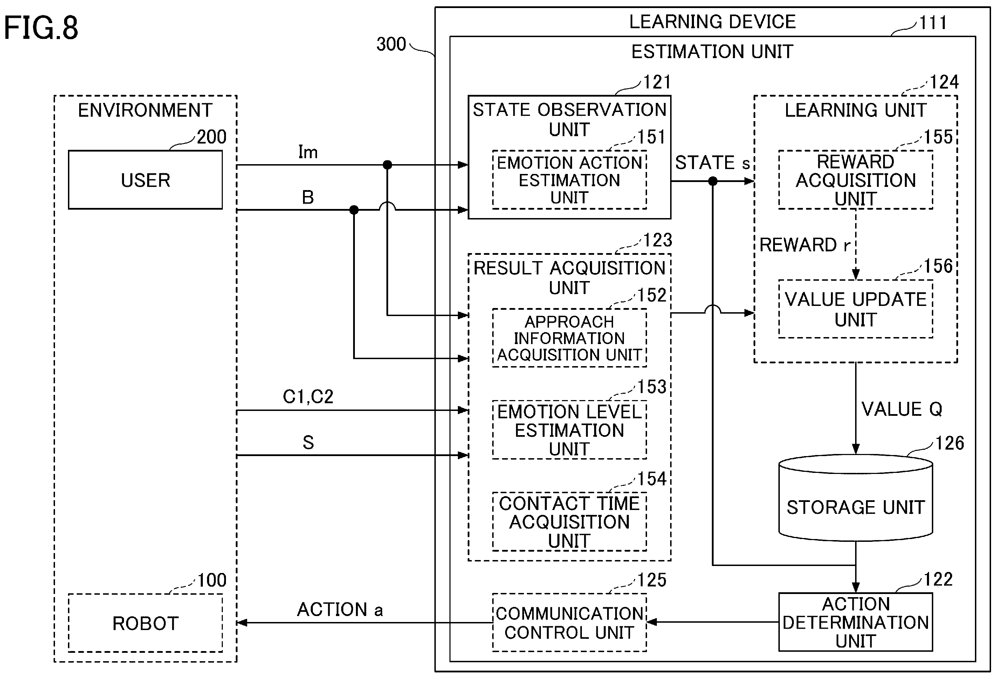

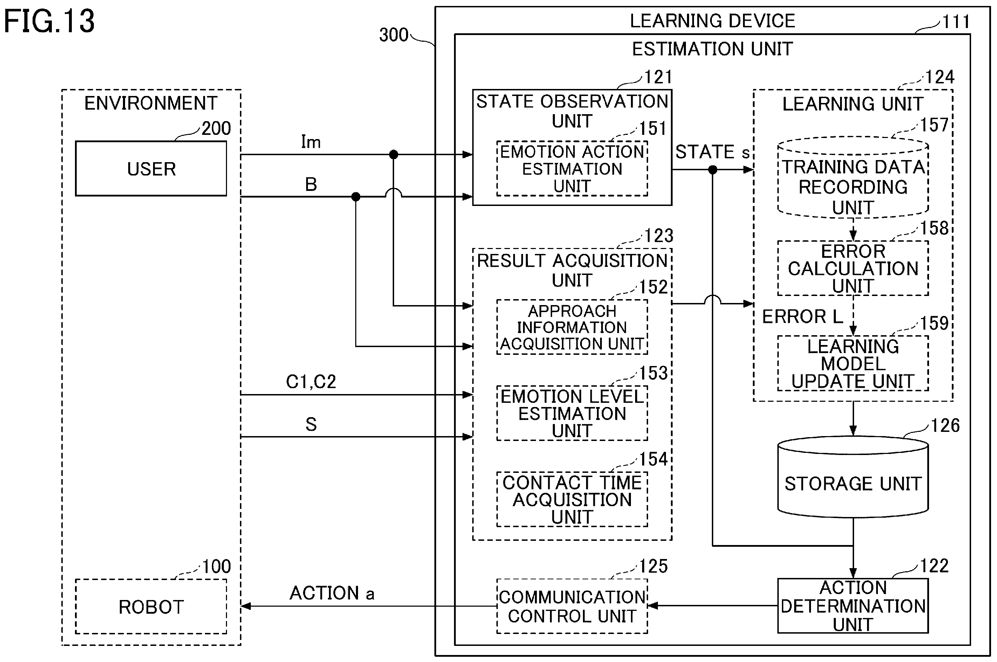

The configuration of the estimation unit 111 that performs reinforcement learning will be described in detail later with reference to FIG. 8. Additionally, the configuration of the estimation unit 111 that performs supervised learning will be described in detail later with reference to FIG. 13. Further, when the learning has converged, the estimation unit 111 may estimate the action at of the robot 100 suitable for the state st of the user 200, using a trained learning model (in the present embodiment, an action value table or a neural network). In this case, the estimation unit 111 estimates a predetermined action at of the robot 100 suitable for the state st of the user 200 by predetermined logic or a predetermined algorithm.

The action control unit 112 instructs the motor control unit 107 or the output unit 108 to perform the action at of the robot 100. Additionally, the action control unit 112 instructs to perform the action at that induces interaction with the user 200 in accordance with the state st of the user 200. The robot 100 performs the action that induces interaction with the user 200 at an appropriate timing, thereby leading to interaction with the user 200 and providing comfort to the user 200.

Here, the action at that induces interaction with the user 200 is an action that explicitly induces interaction with the user 200, such as "spread arms" or "wave a hand", for example. Additionally, the action at that induces interaction with the user 200 may be an action that implicitly induces interaction with the user 200, such as "frightened" or "sing".

The storage unit 103 stores information on a predefined action an of the robot 100. The information on the action an of the robot 100 is managed by, for example, a table of a database. The following table 1 is an example of an action table TB1 related to the action an of the robot 100. The action table TB1 includes an action ID for identifying the action an of the robot 100, an action content of the robot 100, a command content of the action an, the action time per one cycle, and an example of use.

[Table 1]

| ACTION ID | ACTION CONTENT | COMMAND CONTENT | ACTION TIME | EXAMPLE OF USE |
|---|---|---|---|---|
| a0 | SPREAD ARMS (SPREAD ARMS TO MAKE IT EASIER TO HUG) | 35: TAUGHT COMMAND, ROTATIONAL SPEED 100 mm/s | 5 s/cycle | INDUCE EXPLICIT INTERACTION |
| a1 | BE FRIGHTENED (TREMBLE OR PUT HANDS ON HEAD AND LOWER HEAD TO GUARD ITSELF) | 35: TAUGHT COMMAND, ROTATIONAL SPEED 200 mm/s | 3 s/cycle | INDUCE IMPLICIT INTERACTION (REACTION WHEN USER IS ANGRY) |
| a2 | WAVE HAND (RAISE HAND AND WAVE SIDE TO SIDE) | 24: TAUGHT COMMAND, ROTATIONAL SPEED 200 mm/s | 5 s/cycle | INDUCE EXPLICIT INTERACTION |
| a3 | SING (LYRICAL OR NON-LYRICAL) | 25: EMIT SOUND | 10 s/cycle | INDUCE IMPLICIT INTERACTION |

(continued)

| ACTION ID | ACTION CONTENT | COMMAND CONTENT | ACTION TIME | EXAMPLE OF USE |
|---|---|---|---|---|
| a4 | TURN AROUND (MOVE TO LOOK AT PERSON BEHIND) | 35: TAUGHT COMMAND, ROTATIONAL SPEED 100 mm/s | 3 s/cycle | INDUCE IMPLICIT INTERACTION |
| a5 | POINT WITH FINGER (DIRECT ARM OR HAND TOWARD OBJECT OR PERSON) | 35: TRACKING COMMAND + TAUGHT COMMAND, ROTATIONAL SPEED 200 mm/s | 3 s/cycle | INDUCE IMPLICIT INTERACTION |
| a6 | TURN HALFWAY BACK (MOVE AWAY SLIGHTLY AND TURN HALFWAY BACK) | 35: TAUGHT COMMAND, ROTATIONAL SPEED 100 mm/s | 3 s/cycle | INDUCE IMPLICIT INTERACTION |
| a7 | APPROACH (MOVE CLOSER AS IF WANTING TO BE TOUCHED) | 35: TRACKING COMMAND, ROTATIONAL SPEED 100 mm/s | 30 s/cycle | INDUCE EXPLICIT INTERACTION |
| a8 | ACT UNWELL (CROUCH DOWN OR LIE DOWN APPEARING UNWELL) | 35: TAUGHT COMMAND, ROTATIONAL SPEED 200 mm/s | 60 s/cycle | INDUCE IMPLICIT INTERACTION |
| a9 | DANCE (DANCE BY MOVING AT LEAST ONE OF ARM, LEG, OR HEAD) | 35: TAUGHT COMMAND, ROTATIONAL SPEED 200 mm/s | 15 s/cycle | INDUCE IMPLICIT INTERACTION |
| a10 | FLAP HANDS (SPREAD HANDS AND FLAP HANDS UP AND DOWN) | 35: TAUGHT COMMAND, ROTATIONAL SPEED 200 mm/s | 3 s/cycle | INDUCE IMPLICIT INTERACTION |
| a18 | EMIT SOUND (EMIT CALLING SOUND, WORRIED SOUND, SAD SOUND, OR CONFUSED SOUND) | 25: EMIT SOUND | 2 s/cycle | INDUCE EXPLICIT INTERACTION |
| a19 | CHANGE EXPRESSION 1 (CAUSE CHEEK TO FLICKER OR COLOR TO CHANGE) | 26: FLICKER/CHANGE COLOR | 5 s/cycle | INDUCE IMPLICIT INTERACTION |
| a20 | CHANGE EXPRESSION 2 (SMILE OR BECOME TEARY-EYED) | 24: DISPLAY IMAGE | 3 s/cycle | INDUCE IMPLICIT INTERACTION |
| a2l | CHANGE EXPRESSION 3 (OPEN AND CLOSE EYES) | 24: DISPLAY IMAGE (MAY BE COMMAND TO PHYSICALLY MOVE EYELID) | 2 s/cycle | INDUCE IMPLICIT INTERACTION |
| a22 | CHANGE EXPRESSION 4 (CHANGE PUPIL OR IRIS SIZE AND CAUSE EYES TO GLOW RED) | 24: DISPLAY IMAGE | 2 s/cycle | INDUCE IMPLICIT INTERACTION |
| a23 | CHANGE EXPRESSION 5 (DISPLAY OR MOVE NOSE, MOUTH EYEBROW, AND EARS) | 24: DISPLAY IMAGE | 3 s/cycle | INDUCE IMPLICIT INTERACTION |
| a24 | CHANGE EXPRESSION 6 (MOVE EYES TO FOLLOW PERSON) | 24: DISPLAY IMAGE | 10 s/cycle | INDUCE IMPLICIT INTERACTION |
| a25 | CHANGE EXPRESSION 7 (DIS-PLAY TEXT OR SYMBOLS DI-RECTLY IN EYES) | 24: DISPLAY CHARACTER STRING | 3 s/cycle | INDUCE EXPLICIT IN-TERACTION |

(continued)

| ACTION ID | ACTION CONTENT | COMMAND CONTENT | ACTION TIME | EXAMPLE OF USE |
|---|---|---|---|---|
| a26 | TILT HEAD SIDEWAYS | 35: TAUGHT COMMAND, ROTATIONAL SPEED 200 mm/s | 2 s/cycle | INDUCE IMPLICIT INTERACTION |
| a27 | RESPOND WHEN EYES MEET (PERFORM ONE OF ACTIONS WHEN EYES MEET) | DEPENDS ON ACTION | DEPENDS ON AC-TION | INDUCE EXPLICIT IN-TERACTION |
| an | ... | ... | ... | ... |

A symbol in the command content in Table 1 is a symbol representing a control target. Additionally, a taught command is an operation command that is previously taught using a teaching method, such as offline teaching, online teaching, or direct teaching. Additionally, a tracking command is an operation command to track the position and posture of the user 200 based on various sensor information, such as the captured image Im (the distance image).

The motor control unit 107 controls the driving of the servo motor 35 in response to a command to perform the action at of the robot 100 from the action control unit 112. When the action content of the robot 100 is, for example, "spread arms", the motor control unit 107 executes the operation command of "spread arms" taught in advance.

The output unit 108 controls communication between the controller 13 and the display 24 in response to a command to perform the action at from the action control unit 112. When the action content of the robot 100 is, for example, "smile with eyes", the output unit 108 outputs data of a smile image to the right-eye display 24a and the left-eye display 24b.

Additionally, the output unit 108 controls communication between the controller 13 and the speaker 25 in response to a command to perform the action at from the action control unit 112. When the action content of the robot 100 is, for example, "emit a (calling) sound", the output unit 108 outputs a calling sound emit signal to the speaker 25.

Further, the output unit 108 controls communication between the controller 13 and the light 26 in response to a command to perform the action at from the action control unit 112. When the action content of the robot 100 is, for example, "cause the cheeks to flicker", the output unit 108 outputs an on/off signal to switching elements of the right-cheek light 26a and the left-cheek light 26b.

<Configuration of Estimation unit 111> (Hardware Configuration Example)

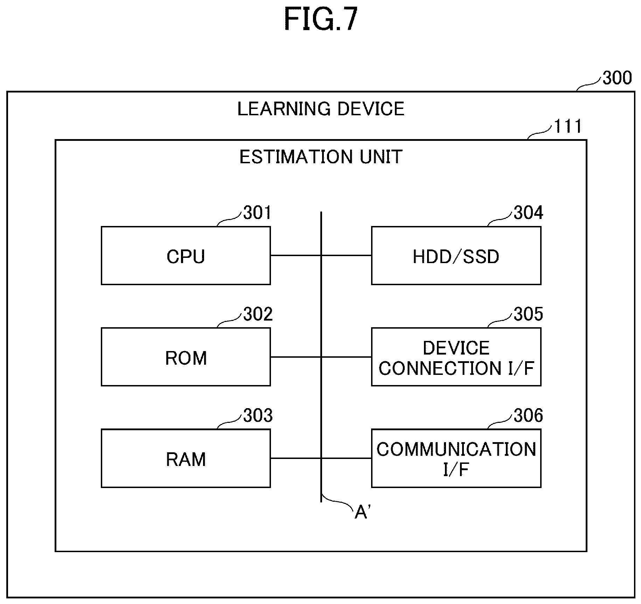

FIG. 7 is a block diagram illustrating a hardware configuration of the estimation unit 111. FIG. 7 illustrates an example in which the estimation unit 111 illustrated in FIG. 6 is configured as a learning device 300 communicably connected to the robot 100. However, the function of the estimation unit 111 may be provided inside the robot 100 as illustrated in FIG. 6.

The estimation unit 111 is constructed by a computer and includes a CPU 301, a ROM 302, a RAM 303, an HDD/SSD 304, a device connection I/F 305, and a communication I/F 306. These are communicably connected to each other via a system bus A'. Here, in order to improve the learning processing capability of the computer, the learning device 300 preferably includes a graphics processing unit (GPU) or is configured by a PC cluster or the like including multiple computers.

The CPU 301 performs control processing including various arithmetic processing. The ROM 302 stores a program used to drive the CPU 301, such as an initial program loader (IPL). The RAM 303 is used as a work area of the CPU 301. The HDD/SSD 304 stores various information, such as a program, the captured image Im acquired by the camera 11, the biological information B acquired by the vital sensor 14, detection information by various sensors, or the like.

The device connection I/F 305 is an interface for connecting the estimation unit 111 to various external devices. The external devices herein are the camera 11, the tactile sensor 12, the vital sensor 14, the first capacitive sensor 21, the second capacitive sensor 31, or the like. However, the estimation unit 111 may acquire the detection information of these various sensors and the like from the robot 100 via the communication I/F 306 to be described later.

The communication I/F 306 is an interface for communicating with an external device, such as the robot 100, via a communication network or the like. For example, the estimation unit 111 is connected to the Internet by the communication I/F 136 and communicates with an external device via the Internet. Additionally, the estimation unit 111 directly communicates with an external device wirelessly through the communication I/F 306.

Here, at least some of the functions realized by the CPU 301 may be realized by an electric circuit or an electronic circuit.

(Functional Configuration Example)

FIG. 8 is a block diagram illustrating a functional configuration of the estimation unit 111. The estimation unit 111 includes a state observation unit 121, an action determination unit 122, a result acquisition unit 123, a learning unit 124, a communication control unit 125, and a storage unit 126. Here, when the function of the estimation unit 111 is provided inside the robot 100, the communication control unit 125 and the storage unit 126 are not necessary. Additionally, when the estimation unit 111 estimates the action at of the robot 100 suitable for the state st of the user 200, using a trained learning model LM or based on a predetermined algorithm, the result acquisition unit 123 and the learning unit 124 are not necessary.

Various functions of the state observation unit 121, the action determination unit 122, the result acquisition unit 123, and the learning unit 124 can be realized by a processor, such as the CPU 301, performing processing defined in a program stored in a non-volatile memory, such as the ROM 302, or the like. Additionally, the function of the communication control unit 125 can be realized by the communication I/F 306 or the like. Further, the function of the storage unit 126 can be realized by a non-volatile memory, such as the HDD/SDD 304.

The estimation unit 111 of the present embodiment estimates the action at of the robot 100 suitable for the state st of the user 200 by performing reinforcement learning. As an algorithm of the reinforcement learning, any one of Q learning, Sarsa, a Monte Carlo method, or deep reinforcement learning (reinforcement learning using DQN (Deep-Q-Network)) can be used. In the following, Q-learning and deep reinforcement learning will be used as examples for the description.

The state observation unit 121 performs various processing for observing the state st of the user 200. The state observation unit 121 observes the state st of the user 200 based on at least one of the captured image Im of the user 200 or the biological information B of the user 200. The captured image Im includes at least one of a face image or a full-body image of the user 200, and the biological information B includes information related to at least one of the heartbeat, the respiration, the blood pressure, or the body temperature of the user 200. For example, the biological information B includes at least one of the heart rate [bpm], the respiratory rate [times/minute], the blood pressure value [mmHg], or the body temperature [°C] of the user 200.

The state st of the user 200 includes an emotional state classified based on at least one of the face image of the user 200 or information on at least one of the heart rate, the respiration, the blood pressure, or the body temperature of the user 200. Additionally, the state st of the user 200 preferably further includes an action state classified based on a motion of a skeleton estimated from the full-body image of the user 200. That is, the state st of the user 200 is a predetermined state classified based on a combination of the emotion and action of the user 200 estimated from at least one of the captured image Im or the biological information B. Here, the state st of the user 200 may be only the emotional state of the user 200 estimated from the captured image Im and the biological information B, or only the action state of the user 200 estimated from the captured image Im of the user 200.

The state observation unit 121 includes an emotion action estimation unit 151. The emotion action estimation unit 151 performs one process in the state observation unit 121. Here, the function of the emotion action estimation unit 151 may be performed by another external device communicably connected to the learning device 300. The emotion action estimation unit 151 estimates the emotion of the user 200 based on at least one of the face image of the user 200 or the information on at least one of the heartbeat, the respiration, the blood pressure, or the body temperature of the user 200. The emotion of the user 200 is classified into a predetermined state based on the face image of the user 200 and the information on at least one of the heartbeat, the respiration, the blood pressure, or the body temperature of the user 200. For example, the emotion of the user 200 is preferably classified in at least one of "neutral", "happiness", "sadness", "disgust", "fear", "surprise", or "anger". The emotion of the user 200 may be a combination of "fear", "surprise", and the like, for example.

The emotion action estimation unit 151 estimates the emotion of the user 200, using a trained learning model or while performing machine learning. For example, the emotion action estimation unit 151 performs, using training data, deep learning on a learning model of a neural network configured to receive the face image, the heart rate, the blood pressure value of the user 200, and the like and output the emotion of the user 200, such as "happiness", "sadness", and "disgust". With this, a new face image, heart rate, and blood pressure value when the user 200 is smiling are input into the learning model of the neural network and analyzed by artificial intelligence (AI), and the emotion of the user 200 can be classified as "happiness".

The emotion action estimation unit 151 estimates the action of the user 200 based on the motion of the skeleton estimated from the full-body image of the user 200. For example, the emotion action estimation unit 151 learns a position of each of the joints from the full-body image of the user 200, estimates the skeleton of the user 200 from the position of each of the joints, and estimates the action of the user 200 based on the motion of the estimated skeleton. The motion of the skeleton is composed of basic motions, such as "stand", "sit", "squat", "walk", "reach out the right arm forward", "reach out the left arm forward", and "shake the head". Additionally, the action of the user 200 is composed of the combination and order of one or more basic motions of the skeleton. For example, the action of the user 200 is classified into at least one of "doing nothing", "walking," "sitting", "operating a mobile phone", "cooking", "typing on a keyboard", "watching television", "sleeping", or the like.

The emotion action estimation unit 151 estimates the action of the user 200, using a trained learning model or while performing machine learning. For example, the emotion action estimation unit 151 performs, using training data, deep learning on a learning model of a first neural network configured to receive a full-body image of the user 200 and output a position of each of the joints of the user 200. Then, the emotion action estimation unit 151 performs, using training data, deep learning on a learning model of a second neural network configured to receive information on a change with respect to the skeleton of the user 200 (a change in the position of each of the joints) and output a basic motion of the skeleton of the user 200. The emotion action estimation unit 151 then estimates the action of the user 200 based on the combination and order of the basic motions of the skeleton. With this, in response to a new full-body image of the user 200 operating a mobile phone being input into the learning model of the neural network and being subjected to AI analysis, the action of the user 200 can be classified as "operating a mobile phone".

The storage unit 126 stores information on a state sn of the user 200 (n is an identification number of the state s). The information on the state sn of the user 200 is managed, for example, by a table in a database. Table 2 below is an example of a state table TB2 related to the state sn of the user 200. The state table TB2 includes a state ID for identifying the state sn of the user 200 and a state content of the user 200. There are the states sn of the user 200 that are equal in number to the number of the combinations of predefined emotions and actions of the user 200. Here, when the function of the estimation unit 111 is provided inside the robot 100, the storage unit 103 of the robot 100 stores the state table TB2.

[Table 2]

| STATE ID | STATE CONTENT |
|----------|---------------|
| s0 | EMOTION: NEUTRAL, ACTION: WALKING |
| s1 | EMOTION: HAPPINESS, ACTION: WALKING |
| s2 | EMOTION: SADNESS, ACTION: WALKING |
| s3 | EMOTION: DISGUST, ACTION: WALKING |
| s4 | EMOTION: FEAR, ACTION: WALKING |
| s5 | EMOTION: SURPRISE, ACTION: WALKING |
| s6 | EMOTION: ANGER, ACTION: WALKING |
| s7 | EMOTION: NEUTRAL, ACTION: SEATED |
| s8 | EMOTION: HAPPINESS, ACTION: SEATED |
| s9 | EMOTION: SADNESS, ACTION: SEATED |
| sn | ... |

For example, when the state st of the user 200 is "emotion: neutral, action: typing on a keyboard", it is observed that the user 200 has a neutral emotion but is busy due to work or the like. Therefore, if the robot 100 performs a certain action at, there is a possibility that it gives stress to the user 200. If the user 200 has a negative emotion, such as "I'm a little busy right now" or "annoying", the user 200 gets tired of the robot 100, and the robot 100 can no longer provide comfort to the user 200.

When the state st of the user 200 is "emotion: happiness, action: sitting", it is observed that the user 200 has a positive emotion and is not very busy. Therefore, if the robot 100 performs an action at that induces interaction with the user 200, the possibility of interaction with the user 200 without giving stress to the user 200 increases. If the opportunities of interaction with the user 200 increase, the robot 100 can provide comfort to the user 200. As described, it is inferred that there is a certain correlation between the state st of the user 200 and a value Q of the action at of the robot 100.

The action determination unit 122 determines the action at of the robot 100 for the state st of the user 200 based on the value Q of the action at-1 (t-1 is the previous time). The storage unit 126 stores an action value table TB3 representing the value Q of the action an of the robot for the state sn of the user 200. Table 3 below is an example of the action value table TB3 at a certain time t.

[Table 3]

|    | a0 | a1 | a2 | a3 | a4 | a5 | a6 | a7 | a8 | a9 | a10 | an |
|----|----|----|----|----|----|----|----|----|----|----|-----|----|
| s0 | +1 | +1 | +1 | +2 | +1 | +1 | +1 | 0  | +2 | +2 | +1  | ... |
| s1 | +2 | +1 | +2 | +3 | +2 | +1 | +1 | +1 | +3 | +3 | +2  | ... |

(continued)

|    | a0 | a1 | a2 | a3 | a4 | a5 | a6 | a7 | a8 | a9 | a10 | an |
|----|----|----|----|----|----|----|----|----|----|----|-----|----|
| s2 | +1 | 0 | 0 | +1 | 0 | 0 | 0 | 0 | +1 | +2 | 0 | ⋯ |
| s3 | 0 | 0 | 0 | 0 | 0 | 0 | 0 | 0 | +1 | 0 | 0 | ⋯ |
| s4 | -1 | 0 | -2 | 0 | +1 | -1 | 0 | 0 | +1 | 0 | 0 | ⋯ |
| s5 | -2 | +1 | -1 | -1 | +2 | -1 | +1 | 0 | +1 | -1 | -1 | ⋯ |
| s6 | -1 | +1 | -2 | -2 | +2 | -1 | +1 | +1 | +1 | -2 | -1 | ⋯ |
| s7 | 0 | 0 | +1 | +1 | 0 | +1 | 0 | 0 | +1 | +2 | +1 | ⋯ |
| s8 | +1 | 0 | +1 | +2 | +1 | 0 | 0 | 0 | +2 | +2 | +1 | ⋯ |
| s9 | 0 | 0 | 0 | 0 | 0 | 0 | 0 | 0 | +1 | 0 | 0 | ⋯ |
| sn | ⋯ | ⋯ | ⋯ | ⋯ | ⋯ | ⋯ | ⋯ | ⋯ | ⋯ | ⋯ | ⋯ | ⋯ |

In an initial state (time t = 0 or the like) of the action value table TB3, the value Q of the action at of the robot 100 for the state st of the user 200 is not known. Therefore, the action determination unit 122 preferably initializes the values Q of all the actions an by random numbers and selects one action at from among the predetermined actions an.

Additionally, when the action determination unit 122 continues to select only the action at having the highest value Q and advances learning, the state does not change to a state st+1 (t+1 is a next timing) that is previously unexperienced. Therefore, the action determination unit 122 previously selects the action at having the highest value Q with the probability 1-ε and selects one action at from among all the actions an with the probability ε, using the ε-greedy method or the like.

For example, when the state st of the user 200 is "emotion: sadness, action: walking", the action determination unit 122 selects an action at: "dance" having the highest value Q with a probability of 0.9 (ε = 0.1). This increases the possibility of inducing interaction with the user 200. Additionally, the action determination unit 122 selects a suitable action at from among all the actions an with a probability of 0.1. This makes the user 200 feel that the robot 100 selects the action at under its free will, and the user 200 does not get bored with the robot 100. Here, when there is no highest value Q and there are multiple same values Q, the action determination unit 122 selects any one action at from among the equal highest values Q by a random number.

The communication control unit 125 transmits, to the robot 100, a command to perform the action at determined by the action determination unit 122. The robot 100 receives the command to perform the action at by the communication control unit 102. Then, the action control unit 112 instructs the motor control unit 107 or the output unit 108 to perform the action at of the robot 100. With this, the robot 100 performs the action at that induces interaction with the user 200 in accordance with the state st of the user 200.

As a result of the action at of the robot 100, the result acquisition unit 123 acquires information on a result of the interaction established with the user 200. The information on the result of the interaction established with the user 200 preferably includes information on the approach of the user 200, information on the emotion of the user 200, and information on the time of the interaction with the user.

The information on the approach of the user 200 includes at least whether the user 200 approaches (whether the user 200 has approached the robot 100). Additionally, the information on the emotion of the user 200 includes at least a positive emotional level or a negative emotional level of the user 200. Furthermore, the time of the interaction with the user 200 includes at least the duration of the interaction with the user 200.

The result acquisition unit 123 includes an approach information acquisition unit 152, an emotional level estimation unit 153, and a contact time acquisition unit 154. At least one of the functions of the approach information acquisition unit 152, the emotional level estimation unit 153, and the contact time acquisition unit 154 may be performed by another external device communicably connected to the learning device 300. Additionally, the function of the emotional level estimation unit 153 may be performed by the emotion action estimation unit 151.

The approach information acquisition unit 152 acquires the information on the approach of the user 200 (whether the user 200 approaches) based on various sensor information, such as the captured image Im (the distance image), the first capacitance signal C1, or the second capacitance signal C2. For example, the approach information acquisition unit 152 acquires information indicating that the user 200 does not approach when the distance from the robot 100 to the user 200 is greater than or equal to a predetermined threshold (e.g., 1 m or greater). Additionally, the approach information acquisition unit 152 acquires information indicating that the user 200 approaches when the distance from the robot 100 to the user 200 is less than the predetermined threshold (e.g., less than 1 m). Furthermore, it is preferable that the approach information acquisition unit 152 further acquires the approaching speed of the user 200 to the robot 100.

The emotional level estimation unit 153 estimates the emotional level of the user 200 based on at least one of the face image of the user 200 or the information on at least one of the heartbeat, the respiration, the blood pressure, or the body temperature of the user 200. For example, the emotional level estimation unit 153 estimates the emotional level as "neutral" when the emotion of the user 200 is classified as "neutral", and estimates the emotional level as "very positive" when the emotion is classified as "happiness". Additionally, the emotional level estimation unit 153 estimates the emotional level as "negative" when the emotion of the user 200 is classified as "sadness", and estimates the emotional level as "very negative" when the emotion is classified as "disgust".

The contact time acquisition unit 154 acquires the information on the time of the interaction with the user 200 based on various sensor information, such as the tactile signal S. For example, the contact time acquisition unit 154 acquires the duration of the interaction with the user 200 by calculating the total time of all ON periods until the tactile signal S is completely turned off in a predetermined period after the robot 100 performs the action at.

As described, as a result of the action at of the robot 100, the result acquisition unit 123 acquires the information on the result of the interaction established with the user 200 (for example, whether the user 200 approaches, the emotional level of the user 200, and the duration of the interaction with the user 200).

The learning unit 124 generates, by reinforcement learning, a learning model LM configured to receive the state st of the user 200 (for example, a combination of the emotion and action) and output the value Q(st, at) of the action at of the robot 100. Additionally, the learning unit 124 updates the learning model LM based on the result of the interaction established with the user 200. In the present embodiment, the learning unit 124 acquires the reward r for the action at of the robot 100 based on the result of the interaction established with the user 200, and updates the value Q (the action value table TB3) of the action at for the state st of the user 200 based on the reward r.

The learning unit 124 includes a reward acquisition unit 155 and a value update unit 156. The reward acquisition unit 155 acquires the reward r for the action at of the robot 100 based on the result of the interaction established with the user 200 (for example, whether the user 200 approaches, the emotional level of the user 200, and the duration of the interaction with the user 200).

The storage unit 126 stores information on a predetermined reward rn (n is an identification number of the reward r) based on the result of the interaction established with the user 200. The information on the reward rn is managed by, for example, a table of a database. Table 4 below is an example of a reward table TB4 indicating the predetermined reward rn. The reward table TB4 includes a reward ID for identifying the reward rn, a result of the interaction established with the user 200, and the reward rn based on the result of the interaction established with the user 200. There are the rewards rn that are equal in number to the number of predefined results of the interaction established with the user 200.

[Table 4]

| REWARD ID | RESULT OF INTERACTION ESTABLISHED WITH USER | REWARD |
|---|---|---|
| r0 | APPROACH: NONE, EMOTIONAL LEVEL: *, INTERACTION DURATION: * | 0 |
| r1 | APPROACH: PRESENT, EMOTIONAL LEVEL: NEUTRAL, INTERACTION DURATION: 1 s to 3 s | +1 |
| r2 | APPROACH: PRESENT, EMOTIONAL LEVEL: POSITIVE, INTERACTION DURATION: 1 s to 3 s | +2 |
| r3 | APPROACH: PRESENT, EMOTIONAL LEVEL: VERY POSITIVE, INTERACTION DURATION: 1 s to 3 s | +3 |
| r4 | APPROACH: PRESENT, EMOTIONAL LEVEL: NEGATIVE, INTERACTION DURATION: 1 s to 3 s | -1 |
| r5 | APPROACH: PRESENT, EMOTIONAL LEVEL: VERY NEGATIVE, INTERACTION DURATION: 1 s to 3 s | -2 |
| r6 | APPROACH: PRESENT, EMOTIONAL LEVEL: NEUTRAL, INTERACTION DURATION: 3 s to 5 s | +2 |
| r7 | APPROACH: PRESENT, EMOTIONAL LEVEL: POSITIVE, INTERACTION DURATION: 3 s to 5 s | +3 |
| r8 | APPROACH: PRESENT, EMOTIONAL LEVEL: VERY POSITIVE, INTERACTION DURATION: 3 s to 5 s | +5 |
| r9 | APPROACH: PRESENT, EMOTIONAL LEVEL: NEGATIVE, INTERACTION DURATION: 3 s to 5 s | +2 |
| rn | … | … |

The reward acquisition unit 155 determines that the interaction with the user 200 is established when the user 200 approaches the robot 100 (the approach: present) and the time of the interaction with the user 200 is greater than or equal to a predetermined threshold (for example, 1 second or greater). In this case, the reward acquisition unit 155 acquires a predetermined reward rn corresponding to the emotional level of the user 200 and the duration of the interaction with the user 200. Although not indicated in Table 4, the reward acquisition unit 155 may acquire a predetermined reward rn corresponding to the approaching speed of the user 200 in addition to the emotional level of the user 200 and the duration of the interaction with the user 200.

When the emotional level of the user 200 is positive, the reward rn may be defined as a positive value that increases as the duration of the interaction with the user 200 becomes longer, and when the configuration of the robot 100 matches the preference of the user 200, it is expected to improve the frequency of the interaction established with the user 200. Additionally, when the emotional level of the user 200 is negative, the reward rn may be defined as a negative value that decreases as the duration of the interaction with the user 200 becomes longer, and when the configuration of the robot 100 matches the preference of the user 200, it is expected to prevent the reduction in the frequency of the interaction established with the user 200.

With respect to the above, the reward acquisition unit 155 determines that the interaction with the user 200 is not established when the user 200 has not approached the robot 100 (the approach: none) or when the duration of the interaction with the user 200 is less than a predetermined time. In this case, the reward acquisition unit 155 acquires a reward rn of zero.

The value update unit 156 updates the value Q of the action at of the robot 100 for the state st of the user 200 based on the predetermined reward rn. In the Q learning, the value Q is updated by the following equation 1.

[Equation 1]

$$Q\left(st,at\right) = Q\left(st,at\right) + \alpha\left(r + \gamma\max Q\left(st+1,at+1\right) - Q\left(st,at\right)\right) \quad \cdots \text{Equation 1}$$

In Equation 1, st is the state of the user 200 at a certain time t, and at is the action of the robot 100 at the certain time t. The action at of the robot 100 changes the state of the user 200 to $st+1$ (t+1 is the next time). $r$ is the reward obtained by the change in the state of the user 200. Additionally, the term with max is obtained by multiplying the value Q by a discount rate $\gamma$ ($0 < \gamma \leq 1$) when the action at+1 with the highest value Q known at that time is selected under the state st+1. Additionally, $\alpha$ is a learning coefficient ($0 < \alpha \leq 1$), and adjusts the learning speed.

Equation 1 indicates a method of updating the value Q(st, at) of the action at for the state st of the user 200 based on the reward r returned as a result of the action at of the robot 100. When a value Q of a certain action at of the robot 100 for a certain state st of the user 200 is less than the sum of the reward r thereof and a discounted value Q of the best action at+1 for the next state st+1, the value Q(st, at) is increased. Conversely, when the value Q(st, at) of the action at in the robot 100 for the state st of the user 200 is greater than the sum of the reward r thereof and the discounted value Q of the best action at+1 for the next state st+1, the value Q(st, at) is decreased. Therefore, Equation 1 attempts to bring the value Q of the certain action at for the certain state st close to the sum of the resultant reward r and the discounted value Q of the best action at+1 for the next state st+1.

The value update unit 156 updates the value Q(sn, an) of the action value table TB3 according to Equation 1. Then, the state observation unit 121 observes the next state st+1 of the user 200. The action determination unit 122 determines the action at+1 of the robot 100 for the next state st+1 of the user 200 by the ε-greedy method or the like based on the value Q of the action at (the action value table TB3 in this example).

The communication control unit 125 transmits, to the robot 100, a command to perform the determined action at+1. The robot 100 receives the command of the action at+1 from the learning device 300 via the communication control unit 102. Then, the action control unit 112 instructs the motor control unit 107 or the output unit 108 to perform the action at+1 of the robot 100. With this, the robot 100 performs the action at+1 suitable for the state st+1 of the user 200.

Here, as a method of expressing the value Q(st, at) on the computer, as described above, there is a method of storing the value Q(st, at) as the action value table TB3 for the combination of all states sn of the user 200 and all actions an of the robot 100. Additionally, there is a method of preparing an action value function that approximates the action value table TB3. The latter method can be realized by adjusting parameters of an approximation function by a method such as a stochastic gradient descent method. For example, as the approximate function, the learning unit 124 preferably generates, by deep reinforcement learning, a learning model of a neural network (DQN) configured to receive the state st (the combination of the emotion and action) of the user 200 and output the value Q of the action at of the robot 100.

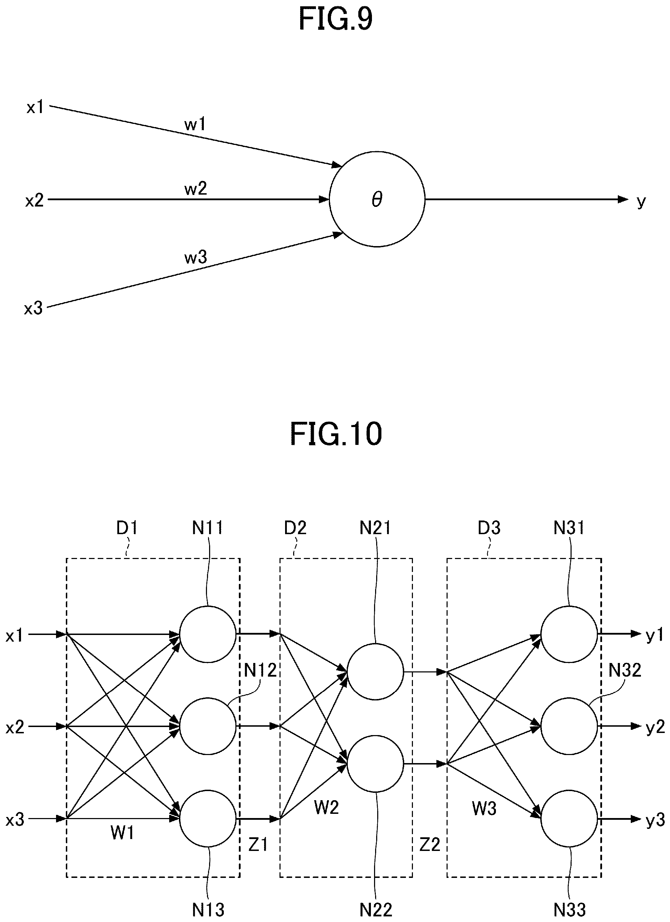

In the following, the deep reinforcement learning will be described, but before that, the neural network will be described. FIG. 9 is a diagram schematically illustrating a learning model of a neuron, and FIG. 10 is a diagram schematically illustrating a learning model of a three-layer neural network configured by combining the neurons illustrated in FIG. 9. The neural network is configured by, for example, an arithmetic device, a memory, and the like that simulate the model of the neuron (a simple perceptron) as illustrated in FIG. 9.

As illustrated in FIG. 9, the neuron outputs an output (result) y with respect to multiple inputs x (in FIG. 9, for example, inputs x1 to x3). Each input x (x1, x2, x3) is multiplied by a weight w (w1, w2, w3) corresponding to the input x. With this, the neuron outputs the output y expressed by the following Equation 2. Here, the input x, the output y, and the weight w are all vectors. Additionally, in the following Equation 2, $\theta$ is a bias, and fk is an activation function.

[Equation 2]

$$y = f_k \left( \sum_{i=1}^{n} x_i w_i - \theta \right) \qquad \cdots \text{Equation 2}$$

FIG. 10 illustrates the three-layer neural network configured by combining the neurons illustrated in FIG. 9. As illustrated in FIG. 10, multiple inputs x (here, for example, the inputs x1 to x3) are input from the left side of the neural network, and the results y (here, for example, the outputs y1 to y3) are output from the right side. Specifically, the inputs x1, x2, and x3 are input into each of three neurons N11 to N13, with corresponding weights being multiplied thereto. The weights multiplied to these inputs are collectively referred to as W1.

The neurons N11 to N13 output z11 to z13, respectively. In FIG. 10, these z11 to z13 are collectively referred to as a feature vector Z1, and can be regarded as a vector obtained by extracting the feature of the input vector. The feature vector Z1 is a feature vector between the weight W1 and the weight W2. z11 to z13 are input into each of the two neurons N21 and N22, with corresponding weights being multiplied thereto. The weights multiplied to the feature vector are collectively referred to as W2.

The neurons N21 and N22 output z21 and z22, respectively. In FIG. 10, these z21 and z22 are collectively referred to as a feature vector Z2. The feature vector Z2 is a feature vector between the weight W2 and the weight W3. z21 and z22 are input into each of the three neurons N31 to N33, with corresponding weights being multiplied thereto. The weights multiplied to the feature vector are collectively referred to as W3.

Finally, the neurons N31 to N33 respectively output outputs y1 to y3. The operation of the neural network includes a learning mode in which the weights W1 to W3 of the neural network are trained and an estimation mode in which the outputs y1 to y3 are estimated from the inputs x1 to x3. For example, in the learning mode, the weights W1 to W3 are trained using a training dataset, and in the estimation mode, the action at of the robot 100 is determined using the parameters. Here, although the term "estimation" is used for convenience, it is needless to say that various tasks, such as detection and classification, can be performed.

Additionally, the weights W1 to W3 can be trained by backpropagation. The error information enters from the right side of the neural network and flows to the left side. The backpropagation is a method of adjusting (training), for each neuron, respective weights so as to reduce a difference (error) between an output y when an input x is input and a true output y (labeled data).

Such a neural network can also perform deep learning by further increasing the number of layers to three or more. Additionally, an arithmetic device including a convolutional neural network (CNN) that extracts features of an input in stages and a neural network that classifies or performs regression on an output can be automatically obtained from training data alone.

In the action value table TB3 described above, when the number of the states sn of the user 200 and the number of actions an of the robot 100 become enormous, the memory space of the action value table TB3 may become too large. Therefore, by approximating the action value table TB3 by a function using a neural network (DQN), an increase in the memory space can be prevented.

The configuration of the estimation unit 111 that performs deep reinforcement learning will be described with reference to FIG. 8 again. The learning unit 124 includes two neural networks (DQN) including a target network TN(value Q(st, at) |θ⁻) and a Q network QN(value Q(st, at) |θ). The two networks are stored in the storage unit 126. The two networks have the same structure, but have different parameters θ (corresponding to the weights described above). The input of each of the two networks is the state st of the user 200, and the output is the value Q(st, at) of the action at of the robot 100.

The state observation unit 121 observes the state st of the user 200 and outputs the state st to the action determination unit 122 and the learning unit 124. The action determination unit 122 determines the action at of the robot 100 by the ε-greedy method or the like, based on the value Q(st, at|θ⁻) of the action at output from the target network TN by inputting the state st of the user 200 into the target network TN. The communication control unit 125 transmits a command to perform the determined action at to the robot 100, and the robot 100 performs the action at in response to the command to perform the action at.

The result acquisition unit 123 acquires a result of the interaction established with the user 200 (whether the user 200 approaches, the emotional level of the user 200, and the duration of the interaction with the user 200) as a result of the action at of the robot 100, and outputs the result to the learning unit 124. The learning unit 124 acquires the reward r based on the result of the interaction established with the user 200. Additionally, the state observation unit 121 observes the next state st+1 of the user 200 and outputs it to the action determination unit 122 and the learning unit 124.

The learning unit 124 stores an experience et(<st, at, st+1, r>) of the robot 100 in the storage unit 126 as an experience buffer. Here, st is the state of the user 200, *at* is the action of the robot 100, *st+1* is the next state of the user 200, and *r* is the reward. Here, the learning unit 124 preferably clips the reward r in a range of -1 to +1 so as not to excessively react to an outlier or the like (i.e., reward clipping).

The learning unit 124 periodically acquires an appropriate experience et from the storage unit 103 (the experience buffer) and causes the Q network QN to be trained. For example, the learning unit 124 acquires experiences (B = e0 to en) for mini-batch learning B from the storage unit 126. Then, the learning unit 124 updates the parameters θ of the Q network QN so as to minimize error L(θ) of a temporal difference (TD) indicated in the following Equation 3 (i.e., experience replay)

[Equation 3]

$$L\left(\theta\right) = \frac{1}{|B|}\sum_{e\in B}(r + \gamma \max Q\left(st+1, at+1 | \theta^-\right) - Q\left(st, at | \theta\right))^2 \quad \cdots \text{Equation 3}$$

Next, the learning unit 124 reflects the parameters θ of the Q network QN in the target network TN at a suitable interval. The learning unit 124 may periodically copy all the parameters θ of the Q network QN to the target network TN, or may reflect the parameters θ of the Q network QN little by little every time the parameters θ of the Q network QN are updated.

The action determination unit 122 inputs the next state st+1 of the user 200 into the target network TN. Then, the action determination unit 122 determines the action at+1 of the robot 100 by the ε-greedy method or the like based on the value Q(st+1, at+1|θ-) of the action at+1 output from the target network TN. The communication control unit 125 transmits, to the robot 100, a command to perform the determined action at+1, and the robot 100 performs the action at+1 in response to the command to perform the action at+1.

As described above, the estimation unit 111 can estimate the action at of the robot 100 suitable for the state st of the user 200 by performing deep reinforcement learning.

<Processing Example by Controller 13>

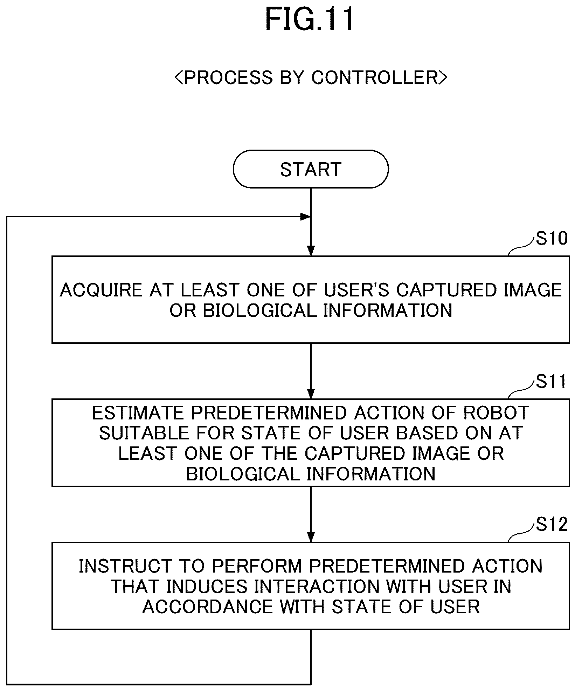

FIG. 11 is a flowchart illustrating a process of the controller 13. FIG. 11 illustrates the process of instructing to perform the action at that induces the interaction with the user 200 in accordance with the state st of the user 200 by the controller 13.

First, in step S10, the controller 13 acquires at least one of the captured image Im of the user 200 or the biological information B of the user 200 by the acquisition unit 101. The captured image Im includes at least one of a face image or a full-body image of the user 200, and the biological information B includes information on at least one of the heartbeat, the respiration, the blood pressure, or the body temperature of the user 200.

Here, in step S10, power is supplied to the camera 11, the tactile sensor 12, the first capacitive sensor 21, the second capacitive sensor 31, and the vital sensor 14 from the battery 15. However, in order to reduce the power consumption of the battery 15, the power is not required to be supplied to the servo motor 35, the display 24, the speaker 25, and the light 26.

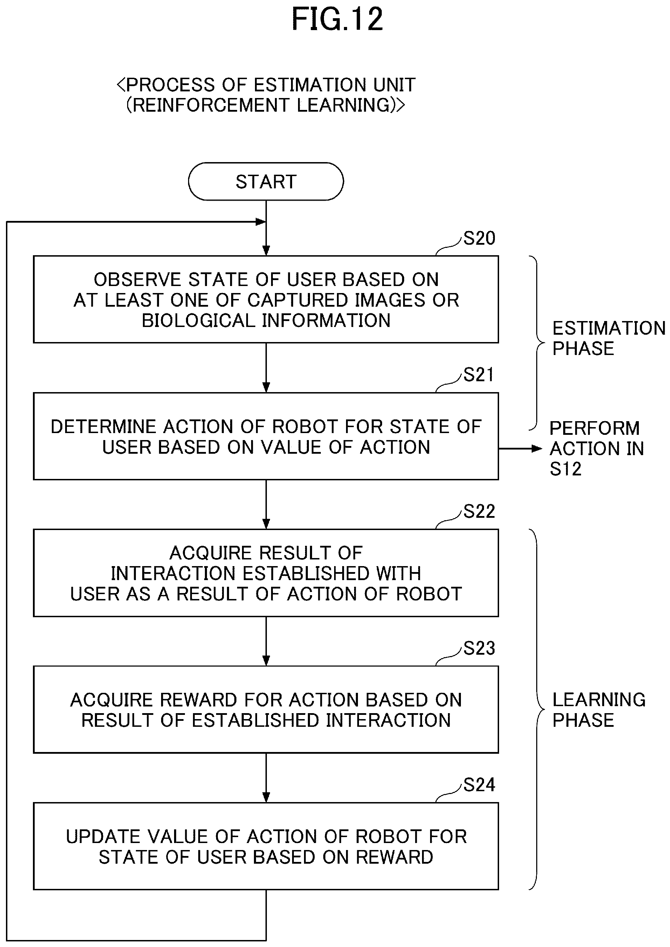

Next, in step S11, the controller 13 estimates the predetermined action at of the robot 100 suitable for the state st of the user 200 based on at least one of the captured image Im or the biological information B by the estimation unit 111. The controller 13 preferably estimates the predetermined action at of the robot 100 suitable for the state st of the user 200 while performing reinforcement learning or using a trained learning model, by the estimation unit 111. Here, the processing in step S11 is not essential processing of the controller 13, and can be performed in an external device (a learning device described later) communicably connected to the robot 100. The detailed processing in step S11 will be described later with reference to FIG. 12.

Then, in step S12, the controller 13 instructs to perform the action at that induces the interaction with the user 200 in accordance with the state st of the user 200 by the action control unit 112. After the robot 100 performs the action at, the controller 13 repeats the processing of steps S10 to S12 to induce the interaction with the user 200, and comfort can be provided to the user 200.

As described above, the controller 13 performs a process of instructing to perform the action at that induces the interaction with the user 200 in accordance with the state st of the user 200. Here, in order to improve the learning processing capability or to suppress the power consumption of the battery 15, when the learning device 300 communicably connected to the robot 100 performs the function of the estimation unit 111, the processing of step S11 is performed by the learning device 300.

Additionally, at the start of the process illustrated in FIG. 11, the servo motor 35, the display 24, the speaker 25, and the light 26 may be in a standby state (a sleep state) in which the supply power amount is reduced. That is, the controller 13 preferably reduces the power consumption of the battery 15 by returning various devices from the standby state in which the supply power is reduced as necessary.

<Process by Estimation Unit 111>

FIG. 12 illustrates a process of the estimation unit 111 (for example, the learning device 300). FIG. 12 illustrates a process in which the estimation unit 111 performs reinforcement learning to estimate the predetermined action at of the robot 100 suitable for the state st of the user 200. Steps illustrated in FIG. 12 are detailed processing of step S11 illustrated in FIG. 11.

First, in step S20, the estimation unit 111 observes the state st of the user 200 based on at least one of the captured image Im or the biological information B by the state observation unit 121. The estimation unit 111 estimates the emotion of the user 200 based on at least one of the face image of the user 200 or the information on at least one of the heartbeat, the respiration, the blood pressure, or the body temperature of the user 200. Additionally, the estimation unit 111 estimates the action of the user 200 based on the motion of the skeleton estimated from the full-body image of the user 200. Then, the estimation unit 111 preferably observes the predetermined state st of the user 200 classified from the combination of the emotion and action of the user 200.

Next, in step S21, the estimation unit 111 determines the action at of the robot 100 suitable for the state st of the user 200 based on the value Q of the action at-1 (the action value table TB3 or the learning model LM, such as DQN described above) by the action determination unit 122. The estimation unit 111 outputs a command to perform the action at to the action control unit 112 or transmits the command to the robot 100 via the communication control unit 125, and thus the robot 100 performs the action at (step S12).

Here, step S20 and step S21 are an estimation phase in which the action at of the robot 100 suitable for the state of the user 200 is estimated, and the other steps are a learning phase.

In step S22, the estimation unit 111 acquires the information on the result of the interaction established with the user 200 as a result of the action at of the robot 100 by the result acquisition unit 123. The result of the interaction established with the user 200 preferably includes, for example, whether the user 200 approaches, the emotional level of the user 200, and the duration of the interaction with the user 200.

In step S23, the estimation unit 111 acquires the reward r for the action at of the robot 100 based on the result of the interaction established with the user 200 by the reward acquisition unit 155.

In step S24, the estimation unit 111 updates the value Q of the action at of the robot 100 for the state st of the user 200 by the value update unit 156 based on the reward r.

After the learning, the process returns to step S20, and the estimation unit 111 observes the next state st+1 of the user 200 by the state observation unit 121. Then, in step S21, the estimation unit 111 determines the action at+1 of the robot 100 suitable for the next state st+1 of the user 200 by the action determination unit 122 based on the updated value Q of the action at. Then, the robot 100 performs the next action at+1 (step S12).

Here, after step S24, the estimation unit 111 may provide a step of determining whether the value Q of the action at has converged (that is, whether the learning has converged) by the value update unit 156. When determining that the learning has converged, the estimation unit 111 does not need to perform the learning phase in the subsequent processing. That is, the estimation unit 111 performs only the estimation phase, and estimates the action at+n of the robot 100 suitable for the state st+n of the user 200, using the trained learning model LM (the action value table TB3, DQN, or the like) (t+n is a timing after n times).

<Configuration of Estimation Unit 111 of Modification Example>

FIG. 13 is a block diagram illustrating a functional configuration of the estimation unit 111 according to a modification example. The estimation unit 111 of the modification example is different from the functional configuration of the estimation unit 111 illustrated in FIG. 8 in that the action at of the robot 100 suitable for the state st of the user 200 is estimated by performing supervised learning. That is, the learning unit 124 includes a training data recording unit 157, an error calculation unit 158, and a learning model update unit 159. In the following, only differences from the configuration of the estimation unit 111 illustrated in FIG. 8 will be described.

The function of the training data recording unit 157 can be realized by a non-volatile memory, such as the HDD/SSD 304. Additionally, the functions of the error calculation unit 158 and the learning model update unit 159 can be realized by a processor, such as the CPU 301, executing processing defined in a program stored in a non-volatile memory, such as the ROM 302, and the like.

The learning unit 124 can use a decision tree (a regression tree), a neural network, logistic regression, or the like as the learning model LM for performing supervised learning. In the following, an example, in which the learning unit 124 generates the learning model LM of the neural network configured to receive the state st of the user 200 and output the value Q of the action at of the robot 100, by supervised learning, will be described.

The training data recording unit 157 stores training data obtained in the past by, for example, another robot 100, simulation, or the like. The training data is data with a result (a label) including a state st-n (t-n is a timing n times before) of the user 200, an action at-n of the robot 100, and a value Q (corresponding to a label) of the action at-n. The estimation unit 111 receives the training data from another robot 100 or another external device via the communication control unit 125 or the like. Additionally, the estimation unit 111 may store experiences experienced by the robot 100 itself as the training data.

The error calculation unit 158 first acquires the training data from the training data recording unit 157 and calculates the error L of the value Q of the action at based on the training data. For example, when the interaction with the user 200 is actually established, the error calculation unit 158 calculates the error L by assuming that there is an error of -log(Q(st, at)). Additionally, when the interaction with the user 200 is not actually established, the error calculation unit 158 calculates the error L by assuming that there is an error of -log(1-Q(st, at)).

The learning model update unit 159 updates the parameters (the above-described weights and the like) of the learning model LM of the neural network so as to minimize the error L. The above-described error back propagation method (back propagation) can be used to update the learning model LM. With this, the learning unit 124 generates the learning model LM that has been trained to a certain level using the training data.

Thereafter, the estimation unit 111 estimates the action at suitable for the actual state st of the user 200, using the learning model LM generated by supervised learning. Then, the robot 100 performs the action at that induces the interaction with the user 200 in accordance with the state st of the user 200.

More specifically, the state observation unit 121 observes the state st of the user 200, and the action determination unit 122 determines the predetermined action at suitable for the state st of the user 200, using the learning model LM. Then, the communication control unit 125 transmits a command to perform the action at to the robot 100, and the robot 100 performs the action at in response to the received command to perform the action at.

The result acquisition unit 123 acquires a result of the interaction established with the user 200 as a result of the action at of the robot 100. The error calculation unit 158 calculates the error of the value Q of the action at based on the result of the interaction established with the user 200, and the learning model update unit 159 further updates the learning model LM of the neural network so as to minimize the error L. Then, the action determination unit 122 determines the action at+1 of the robot 100 suitable for the next state st+1 of the user 200, using the learning model LM.

As described above, the estimation unit 111 can estimate the action of the robot 100 suitable for the state st of the user 200, using the learning model LM trained to a certain level by supervised learning. For example, even when the robot 100 breaks down and is replaced with the robot 100 of the same model number, the robot 100 after the replacement can learn the past experiences based on the training data of the robot that has broken down and can immediately perform the action at suitable for the emotional state st of the user 200. Additionally, for the user 200 with no prior interaction, the robot 100 can perform the action at suitable for the state st of the user 200 to some extent.

<Process by Estimation Unit 111 of Modification Example>

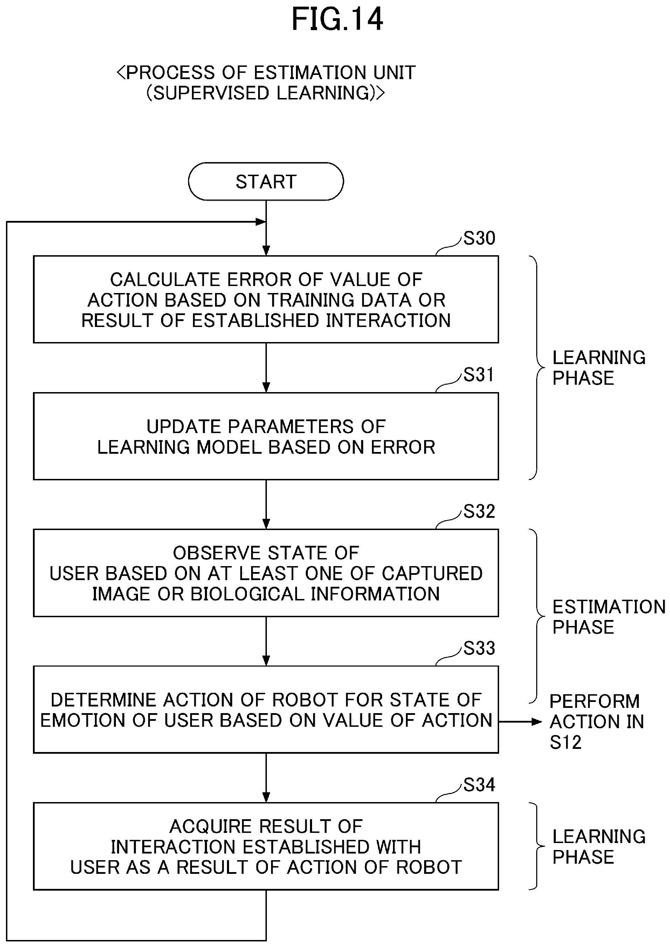

FIG. 14 is a flowchart illustrating a process of the estimation unit 111 (the learning device 300) of the modification example. FIG. 14 illustrates the process in which the estimation unit 111 performs supervised learning to estimate the action at of the robot 100 suitable for the state st of the user 200. Steps illustrated in FIG. 14 are detailed processing of step S11 illustrated in FIG. 11.

First, in step S30, the estimation unit 111 acquires the training data from the training data recording unit 157 and calculates the error L of the value Q of the action at of the robot 100 based on the training data by the error calculation unit 158.

Next, in step S31, the estimation unit 111 updates the parameters (the weights and the like described above) of the learning model LM of the neural network so as to minimize the error L by the learning model update unit 159. With this, the estimation unit 111 can estimate the action at suitable for the actual state st of the user 200, using the learning model LM trained to a certain level by the training data.

Thereafter, in step S32, the estimation unit 111 observes the actual state st of the user 200 based on at least one of the captured image Im or the biological information B by the state observation unit 121. The estimation unit 111 estimates the emotion of the user 200 based on at least one of the face image of the user 200 or the information on at least one of the heartbeat, the respiration, the blood pressure, or the body temperature of the user 200. Additionally, the estimation unit 111 estimates the action of the user 200 based on the motion of the skeleton estimated from the full-body image of the user 200. Then, the estimation unit 111 preferably observes the predetermined state st of the user 200 classified from the combination of the emotion and action of the user 200.

Next, in step S33, the estimation unit 111 determines the action at of the robot 100 suitable for the state st of the user 200 based on the value Q (the learning model LM, such as a neural network) of the action at-1 by the action determination unit 122. The estimation unit 111 outputs a command to perform the action at to the action control unit 112 or transmits the command to the robot 100 via the communication control unit 125. With this, the robot 100 performs the action at corresponding to the state st of the user 200 (step S12).

Here, steps S32 and S33 are the estimation phase for estimating the action at of the robot 100 suitable for the state of the user 200, and the other steps are the learning phase.

In step S34, the estimation unit 111 acquires the information on the result of the interaction established with the user 200 as a result of the action at of the robot 100 by the result acquisition unit 123. The information on the result of the interaction established with the user 200 preferably includes whether the user 200 approaches, the emotional level of the user 200, and the duration of the interaction with the user 200.

Next, the process returns to step S30, and the estimation unit 111 calculates the error L of the value Q of the action at based on the result of the interaction established with the user 200 by the error calculation unit 158.

Then, in step S31, the estimation unit 111 updates the parameters (the weights and the like) of the learning model LM based on the error L by the learning model update unit 159.

After learning, in step S32, the estimation unit 111 observes the next state st+1 of the user 200 by the state observation unit 121. Then, in step S33, the estimation unit 111 determines the action at+1 of the robot 100 suitable for the next state st+1 of the user 200 based on the updated value Q of the action at by the action determination unit 122. Then, the robot 100 performs the next action at+1 (step S12).

Here, after step S31, the estimation unit 111 may provide a step of determining whether the value Q of the action at has converged (that is, whether the learning has converged) by the learning model update unit 159. When determining that the learning has converged, the estimation unit 111 does not need to perform the learning phase in the subsequent processing. That is, the estimation unit 111 performs only the estimation phase, and estimates the action at+n of the robot 100 suitable for the state st+n (t+n is a timing after n times) of the user 200, using the trained learning model LM (the neural network or the like).

<Operation Effect of Present Embodiment>

As described, the robot 100 estimates the predetermined action at suitable for the state st of the user 200 based on at least one of the captured image Im of the user 200 or the biological information B of the user 200. Then, the robot 100 performs the action at that induces the interaction with the user 200 in accordance with the state st of the user 200. Therefore, the robot 100 can induce the interaction with the user 200 at an appropriate timing. Consequently, the interaction with the user 200 is continuously established, and comfort can be continuously provided to the user 200.

Additionally, the captured image Im includes at least one of the face image or the full-body image of the user 200, and the biological information B includes the information on at least one of the heartbeat, the respiration, the blood pressure, or the body temperature of the user 200. The state st of the user 200 can be observed more accurately in comparison with the case in which the state st of the user is observed only from the captured image Im or the biological information B.

In particular, the state st of the user 200 preferably includes the emotion of the user classified based on at least one of the face image of the user 200 or the information on at least one of the heartbeat, the respiration, the blood pressure, or the body temperature of the user 200. The user 200 does not necessarily have a positive emotion, even if the face is temporarily smiling. Therefore, the state st of the user 200 can be observed with high accuracy by considering not only the face image of the user 200 but also the information on at least one of the heartbeat, the respiration, the blood pressure, or the body temperature of the user 200.

Additionally, the state st of the user 200 preferably includes the action of the user 200 classified based on the motion of the skeleton estimated from the full-body image of the user 200. The user 200 may be busy with housework, work, or the like, even if the user has a positive emotion. Therefore, the state st of the user 200 can be observed with high accuracy by observing the state st of the user 200 in consideration of not only the emotion of the user 200 but also the action of the user 200.

Furthermore, the state st of the user is a predetermined state sn classified based on the combination of the emotion and action of the user 200 estimated from at least one of the captured image Im or the biological information B. With this, the states sn of the user 200 are classified into tens to hundreds of types, and thus the increase of the memory space of the action value table TB3 can be suppressed.

Additionally, the robot 100 generates the learning model LM configured to receive the state st of the user 200 and output the value Q of the action at of the robot 100 by machine learning (reinforcement learning, supervised learning, or the like). Therefore, the robot 100 can learn the action at suitable for the state st of the user 200 and performs the action at that induces the interaction with the user 200 at an appropriate timing. Consequently, the interaction with the user 200 is continuously established, and comfort can be continuously provided to the user 200.

Furthermore, the robot 100 further includes the result acquisition unit 123 configured to acquire the information on the result of the interaction established with the user 200, and the learning unit 124 updates the learning model LM based on the result of the interaction established with the user 200. Therefore, the robot 100 can continuously learn the action at suitable for the state st of the user 200 and perform the action at that induces the interaction established with the user 200 at an appropriate timing. Additionally, the learning model LM can increase the reliability of the learning capability of the robot 100 by using the action value table TB3 or the neural network (DQN), the effect of which has already been demonstrated.

Additionally, the information on the result of the interaction established with the user 200 includes whether the user 200 approaches, the emotional level of the user 200, and the duration of the interaction with the user 200. Therefore, the robot 100 can determine whether the interaction with the user 200 has been established based on whether the user 200 approaches and the duration of the interaction with the user 200. Then, based on the result of the interaction established with the user 200, the learning unit 124 acquires the reward r for the action at of the robot 100, and updates the value Q of the action at for the state st of the user based on the reward r. Therefore, the robot 100 can learn the action at suitable for the state st of the user 200 in accordance with the emotional level of the user 200 and the duration of the interaction with the user 200.

Additionally, the robot 100 may estimate the action at of the robot 100 suitable for the state st of the user 200, using the learning model LM trained to a certain level by supervised learning. With this, for example, even when the robot 100 breaks down and is replaced with the robot 100 of the same model number, the robot 100 after the replacement can learn the past experiences based on the training data and immediately perform the action at suitable for the state st of the user 200. Additionally, the robot 100 can perform the action at suitable for the state st of the user 200 to some extent for the user 200 with no prior interaction.

Furthermore, the action an of the robot 100 that induces the interaction with the user 200 includes not only the action that explicitly induces the interaction, but also the action that causes the user 200 to pay attention to the action of the robot 100 or the action that implicitly induces the interaction. Therefore, even in the state st in which the user 200 has a negative emotion or in the state st in which the user 200 is busy, the user 200 is hardly stressed. Rather, the user 200 can have a positive impression that the robot 100 is cute or funny.

The above-described function of the estimation unit 111 of the robot 100 may be provided in the learning device 300 communicably connected to the robot 100 and may be distributed and processed. This can improve the learning processing capability of the computer. Additionally, the distributed processing by the learning device 300 can provide technical effects, such as a reduction in power consumption, a reduction in the number of times of charging, and a reduction in battery weight of the battery 15 of the robot 100.

Although the preferred embodiments have been described in detail above, the present invention is not limited to the above-described embodiments, and various modifications and substitutions can be made to the above-described embodiments without departing from the scope of the appended claims.

Additionally, the numerals, such as ordinal numbers and quantities used in the description of the above-described embodiments are all examples for specifically describing the technique of the present invention, and the present invention is not limited to the numerals described as examples. Additionally, the connection relationship between the constituent elements is an example for specifically describing the technique of the present invention, and the connection relationship for realizing the functions of the present invention is not limited thereto.

The robot according to the present embodiment is particularly suitable for use in promoting oxytocin secretion and providing comfort (sense of security or self-affirmation) to a working adult living alone, a senior person whose child has moved out, a frail elderly person who is a target of home healthcare, or the like. However, the present invention is not limited to this use, and can be used to provide comfort to various users.

Aspects of the present disclosure are as follows, for example.

<1> A robot including:

an acquisition unit configured to acquire at least one of a captured image of a user or biological information of the user; and
an action control unit configured to instruct to perform a predetermined action that induces interaction with the user in accordance with a state of the user based on the at least one of the captured image or the biological information.

<2> The robot as described in <1>, further including an estimation unit configured to estimate the predetermined action suitable for the state of the user,
wherein the estimation unit includes:

a state observation unit configured to observe the state of the user based on the at least one of the captured image or the biological information; and
an action determination unit configured to determine the predetermined action suitable for the state of the user based on a value of the predetermined action.

<3> The robot as described in <1> or <2>,

wherein the captured image includes at least one of a face image or a full-body image of the user, and
wherein the biological information includes information on at least one of a heartbeat, respiration, a blood
pressure, or a body temperature of the user.

<4> The robot as described in any one of <1> to <3>, wherein the state of the user includes an emotion of the user classified based on at least one of a face image of the user or information on at least one of a heartbeat, respiration, a blood pressure, or a body temperature of the user.

<5> The robot as described in any one of <1> to <4>, wherein the state of the user includes an action of the user classified based on a motion of a skeleton estimated from a full-body image of the user.

<6> The robot as described in any one of <1> to <5>, wherein the state of the user is a predetermined state classified based on a combination of an emotion and an action of the user estimated from the at least one of the captured image or the biological information.

<7> The robot as described in <2>, wherein the estimation unit includes a learning unit configured to generate a learning model by machine learning, the learning model being configured to receive the state of the user and output the value of the action of the robot.

<8> The robot as described in <7>,

wherein the estimation unit further includes a result acquisition unit configured to acquire information on a result of the interaction established with the user as a result of the action of the robot, and
wherein the learning unit updates the learning model based on the result of the interaction established with the user.

<9> The robot as described in <7> or <8>, wherein the learning model is an action value table or a neural network.

<10> The robot as described in <8>,

wherein the information on the result of the interaction established with the user includes whether the user approaches, an emotional level of the user, and a duration of the interaction with the user, and
wherein the learning unit acquires a reward for the action of the robot based on the result of the interaction established with the user, and updates the value of the action for the state of the user based on the reward.

<11> A learning device communicably connected to a robot, including:

a state observation unit configured to observe, based on at least one of a captured image of a user or biological information of the user, a state of the user; and
a learning unit configured to generate a learning model by machine learning, the learning model being configured to receive the state of the user and output a value of an action of the robot.

<12> The learning device as described in <11>, further including:

an action determination unit configured to determine the action of the robot suitable for the state of the user based on the value of the action; and
a communication control unit configured to transmit, to the robot, a command to perform the action.

<13> The learning device as described in <11> or <12>, further comprising a result acquisition unit configured to acquire information on a result of interaction established with the user as a result of the action of the robot,
wherein the learning unit updates the learning model based on the result of the established interaction.

<14> A control method of a robot, the control method including:

a step of acquiring, by the robot, at least one of a captured image of a user or biological information of the user; and
a step of performing, by the robot, a predetermined action that induces interaction with the user in accordance with a state of the user based on the at least one of the captured image or the biological information.

<15> A program for causing a computer controlling a robot to perform:

a step of acquiring at least one of a captured image of a user or biological information of the user; and
a step of instructing to perform a predetermined action that induces interaction with the user in accordance with a state of the user based on the at least one of the captured image or the biological information.

This application is based on and claims priority to Japanese Patent Application No. 2022-156758 filed in the Japan Patent Office on September 29, 2022, the entire contents of which are incorporated herein by reference.

Description of reference symbols

1 trunk
2 head
2a right eye
2b left eye
2c mouth
2d right cheek
2e left cheek
3 arm
3a right arm
3b left arm
4 leg
4a right leg
4b left leg
10 exterior member
11 camera
12 tactile sensor
13 controller
14 vital sensor (electromagnetic wave sensor)
141 microwave transmitter
142 microwave receiver
15 battery
16 trunk frame
17 trunk mounting base
21 first capacitive sensor
22 head frame
23 head mounting base
24 display
24a right-eye display
24b left-eye display
25 speaker
26 light
26a right-cheek light
26b left-cheek light
27 head connection mechanism
31 second capacitive sensor
32a right arm frame
32b left arm frame
33 right arm mounting base
34a right arm connection mechanism
34b left arm connection mechanism
35 servo motor
35a right arm servo motor
35b left arm servo motor
35c head servo motor
35d right leg servo motor
35e left leg servo motor
41a right leg wheel
41b left leg wheel
42a right leg frame

42b left leg frame
44a right leg connection mechanism
44b left leg connection mechanism
100 robot
101 acquisition unit
102 communication control unit
103 storage unit
104 authentication unit
105 registration unit
106 start control unit
107 motor control unit
108 output unit
109 registered information
110 detection unit
111 estimation unit
112 action control unit
121 state observation unit
122 action determination unit
123 result acquisition unit
124 learning unit
125 communication control unit
126 storage unit
131 CPU
132 ROM
133 RAM
134 HDD/SSD
135 device connection I/F
136 communication I/F
151 emotion action estimation unit
152 approach information acquisition unit
153 emotional level estimation unit
154 contact time acquisition unit
155 reward acquisition unit
156 value update unit
157 training data recording unit
158 error calculation unit
159 learning model update unit
200 user
300 learning device
301 CPU
302 ROM
303 RAM
304 HDD/SSD
305 device connection I/F
306 communication I/F
A, A' system bus
B biological information
C1 first capacitance signal
C2 second capacitance signal
F1a right shoulder frame
F2a right upper arm frame
F3a right elbow frame
F4a right forearm frame
F1b left shoulder frame
F2b left upper arm frame
F3b left elbow frame
F4b left forearm frame
F1c neck frame

F2c face frame
Im captured image
L error
LM learning model
Ms transmitted wave
Mr reflected wave
M1a right shoulder servo motor
M2a right upper arm servo motor
M3a right elbow servo motor
M4a right forearm servo motor
M1b left shoulder servo motor
M2b left upper arm servo motor
M3b left elbow servo motor
M4b left forearm servo motor
M1c neck servo motor
M2c face servo motor
Q value
S tactile signal
s state
a action
r reward

**Claims**

1. A robot comprising:

   an acquisition unit configured to acquire at least one of a captured image of a user or biological information of the user; and
   an action control unit configured to instruct to perform a predetermined action that induces interaction with the user in accordance with a state of the user based on the at least one of the captured image or the biological information.

2. The robot as claimed in claim 1, further comprising an estimation unit configured to estimate the predetermined action suitable for the state of the user,
   wherein the estimation unit includes:

   a state observation unit configured to observe the state of the user based on the at least one of the captured image or the biological information; and
   an action determination unit configured to determine the predetermined action suitable for the state of the user based on a value of the predetermined action.

3. The robot as claimed in claim 1 or 2,

   wherein the captured image includes at least one of a face image or a full-body image of the user, and
   wherein the biological information includes information on at least one of a heartbeat, respiration, a blood pressure, or a body temperature of the user.

4. The robot as claimed in claim 1 or 2, wherein the state of the user includes an emotional state of the user classified based on at least one of a face image of the user or information on at least one of a heartbeat, respiration, a blood pressure, or a body temperature of the user.

5. The robot as claimed in claim 1 or 2, wherein the state of the user includes an action state of the user classified based on a motion of a skeleton estimated from a full-body image of the user.

6. The robot as claimed in claim 1 or 2, wherein the state of the user is a predetermined state classified based on a combination of an emotion and an action of the user estimated from the at least one of the captured image or the biological information.

7. The robot as claimed in claim 2, wherein the estimation unit includes a learning unit configured to generate a learning model by machine learning, the learning model being configured to receive the state of the user and output the value of the action of the robot.

8. The robot as claimed in claim 7,

   wherein the estimation unit further includes a result acquisition unit configured to acquire information on a result of the interaction established with the user as a result of the action of the robot, and
   wherein the learning unit updates the learning model based on the result of the interaction established with the user.

9. The robot as claimed in claim 7, wherein the learning model is an action value table or a neural network.

10. The robot as claimed in claim 8,

    wherein the information on the result of the interaction established with the user includes whether the user approaches, an emotional level of the user, and a duration of the interaction with the user, and
    wherein the learning unit acquires a reward for the action of the robot based on the result of the interaction established with the user, and updates the value of the action for the state of the user based on the reward.

11. A learning device communicably connected to a robot, comprising:

    a state observation unit configured to observe, based on at least one of a captured image of a user or biological information of the user, a state of the user; and
    a learning unit configured to generate a learning model by machine learning, the learning model being configured to receive the state of the user and output a value of an action of the robot.

12. The learning device as claimed in claim 11, further comprising:

    an action determination unit configured to determine the action of the robot suitable for the state of the user based on the value of the action; and
    a communication control unit configured to transmit, to the robot, a command to perform the action.

13. The learning device as claimed in claim 11 or 12, further comprising a result acquisition unit configured to acquire information on a result of interaction established with the user as a result of the action of the robot, wherein the learning unit updates the learning model based on the result of the established interaction.

14. A control method of a robot, the control method comprising:

    a step of acquiring, by the robot, at least one of a captured image of a user or biological information of the user; and
    a step of performing, by the robot, a predetermined action that induces interaction with the user in accordance with a state of the user based on the at least one of the captured image or the biological information.

15. A program for causing a computer controlling a robot to perform:

    a step of acquiring at least one of a captured image of a user or biological information of the user; and
    a step of instructing to perform a predetermined action that induces interaction with the user in accordance with a state of the user based on the at least one of the captured image or the biological information.

# FIG.1

# FIG.2

# FIG.3

# FIG.4

# FIG.5

ROBOT 100

CAMERA 11

TACTILE SENSOR 12

VITAL SENSOR 14

FIRST CAPACITIVE SENSOR 21

SECOND CAPACITIVE SENSOR 31

CONTROLLER 13

CPU 131

ROM 132

RAM 133

HDD/SSD 134

DEVICE CONNECTION I/F 135

COMMUNI-CATION I/F 136

A

SERVO MOTOR 35

BATTERY 15

DISPLAY 24

SPEAKER 25

LIGHT 26

EP 4 596 070 A1

**FIG.6**

ROBOT — 100

CONTROLLER — 13

CAMERA — 11 | Im
TACTILE SENSOR — 12 | S
VITAL SENSOR — 14 | B
FIRST CAPACITIVE SENSOR — 21 | C1
SECOND CAPACITIVE SENSOR — 31 | C2

ACQUISITION UNIT — 101
COMMUNICATION CONTROL UNIT — 102
STORAGE UNIT — 103
AUTHENTICATION UNIT — 104
Im
REGISTRATION UNIT — 105
REGISTERED INFORMATION — 109
START CONTROL UNIT — 106
MOTOR CONTROL UNIT — 107
OUTPUT UNIT — 108
DETECTION UNIT — 110
ESTIMATION UNIT — 111
ACTION CONTROL UNIT — 112

BATTERY — 15
SERVO MOTOR — 35
DISPLAY — 24
SPEAKER — 25
LIGHT — 26

EP 4 596 070 A1

# FIG.7

300

## LEARNING DEVICE

111

### ESTIMATION UNIT

| 301 | | 304 |
|---|---|---|
| CPU | | HDD/SSD |

| 302 | | 305 |
|---|---|---|
| ROM | | DEVICE CONNECTION I/F |

| 303 | | 306 |
|---|---|---|
| RAM | | COMMUNICATION I/F |

A'

# FIG.8

ENVIRONMENT

USER ~200

ROBOT ~100

---

LEARNING DEVICE ~111

ESTIMATION UNIT ~300

STATE OBSERVATION UNIT ~121
EMOTION ACTION ESTIMATION UNIT ~151

RESULT ACQUISITION UNIT ~123
APPROACH INFORMATION ACQUISITION UNIT ~152
EMOTION LEVEL ESTIMATION UNIT ~153
CONTACT TIME ACQUISITION UNIT ~154

LEARNING UNIT ~124
REWARD ACQUISITION UNIT ~155
VALUE UPDATE UNIT ~156
REWARD $r$

STATE $s$

STORAGE UNIT ~126
VALUE Q

ACTION DETERMINATION UNIT ~122

COMMUNICATION CONTROL UNIT ~125

ACTION $a$

Im

B

C1,C2

S

EP 4 596 070 A1

# FIG.9

# FIG.10

# FIG.11

<PROCESS BY CONTROLLER>

START

S10

ACQUIRE AT LEAST ONE OF USER'S CAPTURED IMAGE OR BIOLOGICAL INFORMATION

S11

ESTIMATE PREDETERMINED ACTION OF ROBOT SUITABLE FOR STATE OF USER BASED ON AT LEAST ONE OF THE CAPTURED IMAGE OR BIOLOGICAL INFORMATION

S12

INSTRUCT TO PERFORM PREDETERMINED ACTION THAT INDUCES INTERACTION WITH USER IN ACCORDANCE WITH STATE OF USER

# FIG.12

<PROCESS OF ESTIMATION UNIT
(REINFORCEMENT LEARNING)>

START

S20

OBSERVE STATE OF USER BASED ON
AT LEAST ONE OF CAPTURED IMAGES OR
BIOLOGICAL INFORMATION

S21

DETERMINE ACTION OF ROBOT FOR STATE OF
USER BASED ON VALUE OF ACTION

ESTIMATION
PHASE

PERFORM
ACTION IN
S12

S22

ACQUIRE RESULT OF
INTERACTION ESTABLISHED WITH
USER AS A RESULT OF ACTION OF ROBOT

S23

ACQUIRE REWARD FOR ACTION BASED ON
RESULT OF ESTABLISHED INTERACTION

LEARNING
PHASE

S24

UPDATE VALUE OF ACTION OF ROBOT FOR
STATE OF USER BASED ON REWARD

# FIG.13

EP 4 596 070 A1

# FIG.14

<PROCESS OF ESTIMATION UNIT
(SUPERVISED LEARNING)>

```
                    ┌─────────────┐
                    │    START    │
                    └─────────────┘
                           │
  ┌──────────────────────────────────────────────────┐  S30
  │       CALCULATE ERROR OF VALUE OF                 │  ╮
  │   ACTION BASED ON TRAINING DATA OR                │  │
  │   RESULT OF ESTABLISHED INTERACTION               │  │
  └──────────────────────────────────────────────────┘  │ LEARNING
                           │                             │ PHASE
  ┌──────────────────────────────────────────────────┐  S31
  │           UPDATE PARAMETERS OF                    │  │
  │   LEARNING MODEL BASED ON ERROR                   │  │
  └──────────────────────────────────────────────────┘  ╯
                           │
  ┌──────────────────────────────────────────────────┐  S32
  │              OBSERVE STATE OF                     │  ╮
  │  USER BASED ON AT LEAST ONE OF CAPTURED           │  │
  │   IMAGE OR BIOLOGICAL INFORMATION                 │  │ ESTIMATION
  └──────────────────────────────────────────────────┘  │ PHASE
                           │                             │
  ┌──────────────────────────────────────────────────┐  S33
  │  DETERMINE ACTION OF ROBOT FOR STATE OF           │  ╯  PERFORM
  │ EMOTION OF USER BASED ON VALUE OF ACTION          │ ──→ ACTION IN
  └──────────────────────────────────────────────────┘     S12
                           │
  ┌──────────────────────────────────────────────────┐  S34
  │             ACQUIRE RESULT OF                     │  ╮
  │   INTERACTION ESTABLISHED WITH                    │  │ LEARNING
  │   USER AS A RESULT OF ACTION OF ROBOT             │  │ PHASE
  └──────────────────────────────────────────────────┘  ╯
```

EP 4 596 070 A1

INTERNATIONAL SEARCH REPORT

| | International application No. |
| | PCT/JP2023/035067 |

### A. CLASSIFICATION OF SUBJECT MATTER

***A63H 11/00***(2006.01)i; ***B25J 13/08***(2006.01)i
FI: A63H11/00 Z; B25J13/08 A

According to International Patent Classification (IPC) or to both national classification and IPC

### B. FIELDS SEARCHED

Minimum documentation searched (classification system followed by classification symbols)

A63H11/00; B25J9/00-9/22; B25J13/00-13/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 1998-289006 A (YAMAHA MOTOR CO., LTD.) 27 October 1998 (1998-10-27) paragraphs [0006]-[0018], [0033], fig. 2 | 1-15 |
| Y | WO 2017/199662 A1 (GROOVE X, INC.) 23 November 2017 (2017-11-23) paragraphs [0049]-[0055], [0101]-[0103] | 1-15 |
| Y | JP 2021-19966 A (LIVING ROBOT INC.) 18 February 2021 (2021-02-18) paragraphs [0020]-[0021], [0082]-[0098] | 1-15 |
| A | JP 2016-12340 A (SOFTBANK CORP.) 21 January 2016 (2016-01-21) entire text, all drawings | 1-15 |
| A | US 2020/0009739 A1 (LG ELECTRONICS INC.) 09 January 2020 (2020-01-09) whole document | 1-15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 October 2023** | **07 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/035067**

| Patent document cited in search report | | | Publication date (day/month/year) | Patent family member(s) | | | Publication date (day/month/year) |
|---|---|---|---|---|---|---|---|
| JP | 1998-289006 | A | 27 October 1998 | US | 2001/0001318 | A1 | |
| | | | | paragraphs [0027]-[0087], [0121]-[0126], fig. 2 | | | |
| | | | | US | 6175772 | B1 | |
| WO | 2017/199662 | A1 | 23 November 2017 | US | 2019/0077021 | A1 | |
| | | | | paragraphs [0089]-[0098], [0182]-[0186] | | | |
| | | | | GB | 2564821 | A | |
| | | | | DE | 112017002589 | T | |
| | | | | CN | 109070332 | A | |
| JP | 2021-19966 | A | 18 February 2021 | US | 2022/0266161 | A1 | |
| | | | | paragraphs [0031]-[0032], [0094]-[0110] | | | |
| | | | | WO | 2021/020490 | A1 | |
| | | | | TW | 202112506 | A | |
| JP | 2016-12340 | A | 21 January 2016 | JP | 2016-12341 | A | |
| | | | | JP | 2016-12342 | A | |
| | | | | JP | 2016-12914 | A | |
| US | 2020/0009739 | A1 | 09 January 2020 | KR | 10-2019-0100090 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009104878 A

- JP 2022156758 A